(19) Europäisches Patentamt
European Patent Office
Office européen des brevets

(11) Publication number: 0 156 286
A2

(12) # EUROPEAN PATENT APPLICATION

(21) Application number: 85103156.7

(22) Date of filing: 19.03.85

(51) Int. Cl.⁴: C 07 D 417/12
C 07 D 285/00, A 61 K 31/54

(30) Priority: 27.03.84 GB 8407896
28.03.84 GB 8408011

(43) Date of publication of application:
02.10.85 Bulletin 85/40

(84) Designated Contracting States:
AT BE CH DE FR GB IT LI LU NL SE

(71) Applicant: HOECHST U.K. LIMITED
Hoechst House Salisbury Road
Hounslow Middlesex TW4 6JH(GB)

(72) Inventor: Ross, Barry Clive, Dr.
41 Blanford Avenue
Luton, Bedfordshire(GB)

(72) Inventor: Allen, Robert Manning
6 Dormans Close Milton Keynes Village
Milton Keynes Buckinghamshire(GB)

(72) Inventor: Cousins, Simon John
1 Gleman Close Grenleys
Milton Keynes Buckinghamshire(GB)

(74) Representative: Beck, Bernhard et al,
HOECHST AKTIENGESELLSCHAFT Zentrale
Patentabteilung Postfach 80 03 20
D-6230 Frankfurt/Main 80(DE)

(54) **Thiatrizine derivatives.**

(57) Salts of 3-amino-5-[2-[ (2-guanidino-4-thiazolyl) -methylthio] ethylamino] -4-methyl-1,2,4,6-thiatriazine-1, 1-dioxide with various acids are prepared in crystalline form. The salts contain varying amounts of crystal water or crystal solvent.

EP 0 156 286 A2

Croydon Printing Company Ltd

The present invention relates to 3-amino-5-[2-[(2-guanidino-4-thiazolyl)-methylthio]-ethylamino]-4-methyl-1,2,4,6-thiatriazine 1,1-dioxide and derivatives thereof, to processes for their preparation, and to pharmaceutical preparations comprising them.

Our British Patent Application No. 2 129 426A relates to thiatriazine derivatives and describes, inter alia, 3-amino-5-[2-[(2-guanidino-4-thiazolyl)-methylthio]-ethylamino]-4-methyl-1,2,4,6-thiatriazine 1,1-dioxide and the hydrobromide thereof.

3-Amino-5-[2-[(2-guanidino-4-thiazolyl)-methylthio]-ethylamino]-4-methyl-1,2,4,6-thiatriazine 1,1-dioxide is a potent histamine H-2 antagonist, and may be used to treat those conditions resulting from stimulation by histamine of H-2 receptors, either alone, for example in inhibiting gastric acid secretion and thus treating its sequelae, for example, gastric and peptic ulcers; or together wih H-1 antagonists, for example, in allergic and certain inflammatory conditions.

The present invention provides salts of 3-amino-5-[2-[(2-guanidino-4-thiazolyl)-methylthio]-ethylamino]-4-methyl-1,2,4,6-thiatriazine 1,1-dioxide with inorganic and organic acids (with the exception of the hydrobromide salt), especially physiologically tolerable salts. Particularly useful salts with inorganic acids are those with sulphuric acid and with hydrochloric acid; and with organic acids are those with a dicarboxylic acid, for example, with maleic, fumaric, tartaric, oxalic or succinic acid. Of these, the salts with hydrochloric acid and succinic acid are particularly preferred.

The present invention also provides 3-amino-5-[2-[(2-guanidino-4-thiazolyl)methylthio]-ethylamino]-4-methyl-1,2,4,6-thiatriazine 1,1-dioxide or a salt thereof (with the exception of the hydrobromide), in crystalline form.

Salts of 3-amino-5-[2-[(2-guanidino-4-thiazolyl)-

methylthio]ethylamino]-4-methyl-1,2,4,6-thiatriazine 1,1-dioxide are useful in its production, and the physiologically tolerable salts are useful in the formulation of pharmaceutical preparations. For large scale production, it is particularly advantageous to be able to obtain the free base (3-amino-5-[2-[(2-guanidino-4-thiazolyl)-methylthio]-ethylamino]-4-methyl-1,2,4,6-thiatriazine 1,1-dioxide itself) and salts thereof in a crystalline form, particularly a highly crystalline form, as isolation of the desired substance can be carried out easily, for example, by filtration or centrifugation, and purification can be carried out by recrystallisation. For use in pharmaceutical preparations, 3-amino-5-[2-[(2-guanidino-4-thiazolyl)methylthio]ethylamino]-4-methyl-1,2,4,6-thiatriazine 1,1-dioxide and physiologically tolerable salts thereof must fulfil the exacting standards relating to purity that are laid down by the licensing authorities, and the ability to produce 3-amino-5-[2-[(2-guanidino-4-thiazolyl)-methylthio]-ethylamino]-4-methyl-1,2,4,6-thiatriazine 1,1-dioxide and physiologically tolerable salts thereof in a highly crystalline form assists the achievement of such standards. Crystalline forms of 3-amino-5-[2-[(2-guanidino-4-thiazolyl)-methylthio]-ethylamino]-4-methyl-1,2,4,6-thiatriazine 1,1-dioxide and its physiologically tolerable salts are also easier to handle than amorphous forms, particularly in the producion of pharmaceutical preparations, for example, tablets.

In particular, the invention provides 3-amino-5-[2-[(2-guanidino-4-thiazolyl)methylthio]-ethylamino]-4-methyl-1,2,4,6-thiatriazine 1,1-dioxide and the following salts, each in crystalline form. In each case the substance may be characterised by the details of the infra-red (IR) spectrum given, and/or by its X-ray powder diffraction pattern, if given. (For details of the methods used to determine the IR and X-ray data, see the Examples section.)

A1. 3-Amino-5-[2-[(2-guanidino-4-thiazolyl)methylthio]-ethylamino]-4-methyl-1,2,4,6-thiatriazine 1,1-dioxide dihydrate:

IR Spectrum (KBr disc):

$\nu_{max}$ 3480, 3380, 3600-2500 (very broad), 1640 (very broad), 1615, 1580, 1545, 1520, 1500, 1455, 1410, 1330, 1260, 1220, 1190, 1160, 1130, 1085, 1060, 1000, 850, 710 and 670 $cm^{-1}$.

A2. 3-Amino-5-[2-[(2-guanidino-4-thiazolyl)-methylthio]-ethylamino]-4-methyl-1,2,4,6-thiatriazine 1,1-dioxide monohydrate:

IR Spectrum (KBr disc):

$\nu_{max}$ 3580, 3420, 3340, 3180, 1660, 1610, 1570, 1530, 1520, 1455, 1410, 1365, 1340, 1270, 1250, 1220, 1190, 1130, 1120, 1075, 1010, 990, 970, 870, 860, 730, 700 and 660 $cm^{-1}$.

X-Ray Diffraction Pattern (given as d(Å), I for each value):

2.18 vw; 2.29 vw; 2.38 vw; 2.58 vw; 2.64 vw; 2.88 vw; 2.97 vw; 3.05 w; 3.17 w; 3.25 w; 3.33 w; 3.42 w; 3.50 w; 3.72 m; 4.07 m; 4.36 m; 5.10 m; 6.32 m; 6.74 s; 7.39 m.

B. 3-Amino-5-[2-[(2-guanidino-4-thiazolyl)methylthio]-ethylamino]-4-methyl-1,2,4,6-thiatriazine 1,1-dioxide hydrochloride

IR Spectrum:

$\nu_{max}$ 3450-2500 (very broad), 3390, 3280, 3180, 3100, 3060, 1695, 1665, 1610, 1560, 1525, 1485, 1460, 1435, 1410, 1335, 1260, 1215, 1190, 1150, 1130, 1085, 1050, 980, 860, 720 and 650 $cm^{-1}$.

X-Ray Diffraction Pattern given as d(Å), I for each value):

2.53 vw; 2.69 w; 2.83 w; 2.89 m; 2.94 m; 3.29 m; 3.52 m;

3.61 m; 3.92 s; 4.23 w; 4.38 s; 4.71 m; 5.24 m.

C. 3-Amino-5-[2-[(2-guanidino-4-thiazolyl)methylthio]-ethylamino]-4-methyl-1,2,4,6-thiatriazine 1,1-dioxide hemisuccinate

IR Spectrum:

$\nu_{max}$ 3600-2500 (very broad), 1705, 1655 (sh), 1640 (sh), 1620, 1560-1530 (broad), 1490, 1460, 1440, 1405, 1385, 1330, 1240, 1220, 1195, 1150, 1120, 1090, 1060, 985, 855, 805, 715 and 660 $cm^{-1}$.

X-Ray Diffraction Pattern (given as d(Å), I for each value): 2.39 vw; 3.12 vw; 3.39 vw; 3.46 s; 3.62 m; 4.01 m; 4.11 m; 4.69 m; 5.26 w; 5.97 m; 6.76 m; 7.20 m; 7.99 m.

D. 3-Amino-5-[2-[(2-guanidino-4-thiazolyl)methylthio]-ethylamino]-4-methyl-1,2,4,6-thiatriazine 1,1-dioxide sulphate

IR Spectrum (KBr disc):

$\nu_{max}$ 3200-2500 (very broad), 1705, 1660, 1620, 1570, 1530, 1460, 1440, 1410, 1340, 1280, 1230, 1215, 1190, 1130, 1070, 1010, 990, 860, 810 and 715 $cm^{-1}$.

E. 3-Amino-5-[2-[(2-guanidino-4-thiazolyl)methylthio]-ethylamino]-4-methyl-1,2,4,6-thiatriazine 1,1-dioxide maleate

IR Spectrum (Nujol mull):

$\nu_{max}$ 3330 (broad), 3180, 3110, 1700, 1660, 1635, 1610, 1560, 1535, 1400, 1340, 1270, 1235, 1215, 1130, 1120, 1075, 1005, 995, 890, 865, 835, 750 and 720 $cm^{-1}$.

F. 3-Amino-5-[2-[(2-guanidino-4-thiazolyl)methylthio]-ethylamino]-4-methyl-1,2,4,6-thiatriazine 1,1-dioxide hemi-oxalate

IR Spectrum (Nujol mull):

$\nu_{max}$ 3400 (broad), 3200 (broad), 1710, 1660, 1620, 1560, 1540, 1420, 1345, 1305, 1270 (broad), 1235, 1215, 1130

(broad), 1070, 1050, 1000, 980, 870, 840, 765, 725 and 715 $cm^{-1}$.

G. 3-Amino-5-[2-[(2-guanidino-4-thiazolyl)methylthio]-ethylamino]-4-methyl-1,2,4,6-thiatriazine 1,1-dioxide hemifumarate

IR Spectrum (Nujol mull):

$\nu_{max}$ 3500, 3405, 3340, 3220 (broad), 1700, 1640, 1620, 1560, 1530, 1405, 1330, 1270, 1225, 1160, 1130, 1090, 1060, 985, 880, 855, 825, 800, 755, 720 and 680 $cm^{-1}$.

H. 3-Amino-5-[2-[(2-guanidino-4-thiazolyl)methylthio]-ethylamino]-4-methyl-1,2,4,6-thiatriazine 1,1-dioxide tartarate

IR Spectrum (Nujol mull):

$\nu_{max}$ 3400, 3300 (broad), 3210, 1690, 1620, 1565, 1535 (sh), 1400, 1330, 1270, 1240, 1210, 1145, 1120, 1060, 1010, 990, 890, 860, 800 and 720 $cm^{-1}$.

I. 3-Amino-5-[2-[(2-guanidino-4-thiazolyl)methylthio]-ethylamino]-4-methyl-1,2,4,6-thiatriazine 1,1-dioxide picrate

IR Spectrum (Nujol mull):

$\nu_{max}$ 3420, 3160 (broad), 1680, 1650, 1615, 1570, 1555, 1340, 1320, 1280, 1220, 1150 (broad), 1080, 980, 940, 910, 850, 790 and 720 $cm^{-1}$.

3-Amino-5-[2-[(2-guanidino-4-thiazolyl)methylthio]-ethylamino]-4-methyl-1,2,4,6-thiatriazine 1,1-dioxide and its salts are generally obtained in the form of a solvate, and the nature of the solvate and the degree of solvation depend, for example, on the nature of the solvent or solvent mixture and the reaction conditions used. 3-Amino-5-[2-[(2-guanidino-4-thiazolyl)methylthio]-ethylamino]-4-methyl-1,2,4,6-thiatriazine 1,1-dioxide is often obtained initially in the form of the dihydrate, but recrystallisation from water generally yields the

monohydrate. The different solvated forms may or may not be apparent from IR data, but can generally be distinguished by microanalysis. The data given above may apply to only one solvated form of a salt, or may apply to more than one form. The present invention includes all solvated (including hydrated) forms of 3-amino-5-[2-[(2-guanidino-4-thiazolyl)methylthio]ethylamino]-4-methyl-1,2,4,6-thiatriazine 1,1-dioxide and salts thereof, in particular, 3-amino-5-[2-[(2-guanidino-4-thiazolyl)-methylthio]ethylamino]-4-methyl-1,2,4,6-thiatriazine 1,1-dioxide and salts thereof described in A to I above, in any solvated form described above and also in any other solvated form. Solvated forms may comprises more than one type of solvent.

The present invention also provides a process for the production of a salt of 3-amino-5-[2-[(2-guanidino-4-thiazolyl)methylthio]ethylamino]-4-methyl-1,2,4,6-thiatriazine 1,1-dioxide, which comprises reacting 3-amino-5-[2-[(2-guanidino-4-thiazolyl)methylthio]ethylamino]-4-methyl-1,2,4,6-thiatriazine 1,1-dioxide with an acid. Methods for producing salts of free bases are well known. (The term "known" is used herein to mean in actual use in the art or described in the literature of the art.)

The present invention also provides a process for the production of 3-amino-5-[2-[(2-guanidino-4-thiazolyl)-methylthio]ethylamino]-4-methyl-1,2,4,6-thiatriazine 1,1-dioxide and of salts thereof, in crystalline form, which comprises

a)i) reacting a 1,2,4,6-thiatriazine of the general formula A

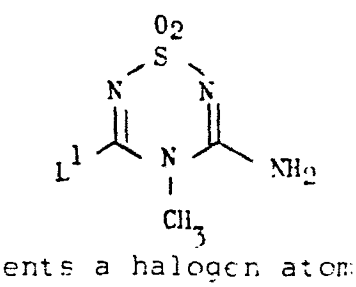

A

in which $L^1$ represents a halogen atom, an alkoxy or phenyloxy group, an alkylthio or phenylthio group, or an

alkylsulphonyl or phenylsulphonyl group, with a compound of the formula B

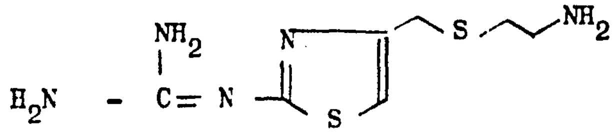

B

a)ii) reacting a 1,2,4,6-thiatriazine of the general formula C

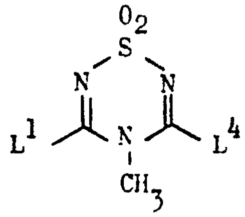

C

in which $L^1$ and $L^4$, which may be the same or different, each represents a halogen atom, an alkoxy or phenyloxy group, an alkylthio or phenylthio group, or an alkylsulphonyl or phenylsulphonyl group, and $L^4$ may also represent a mono- or di- lower alkyl-substituted amine group, with a compound of formula B as defined above, and reacting the resulting compound of formula D

D

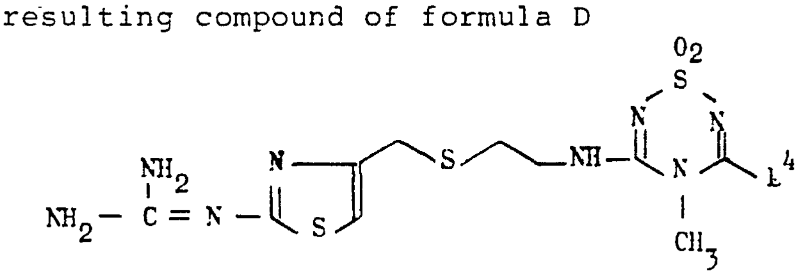

with ammonia; or

a)iii) reacting a 1,2,4,6-thiatriazine of the formula A above with a compound of formula E

$$HS - CH_2 - CH_2 - NH_2 \qquad E$$

and reacting the resulting compound of formula F

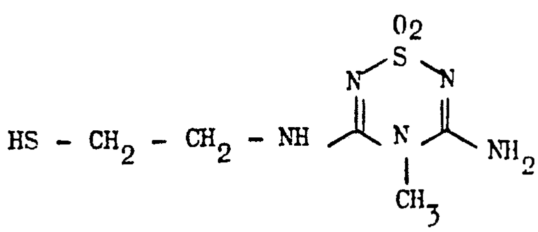

F

with a compound of the general formula G

G

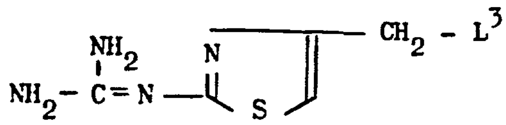

in which $L^3$ represents a leaving group, for example, a halogen atom, a hydroxy group, an alkoxy group, or a sulphonate ester, and isolating the resulting crystalline 3-amino-5-[2-[(2-guanidino-4-thiazolyl)methylthio]ethylamino]-4-methyl-1,2,4,6-thiatriazine-1,1-dioxide from the reaction mixture; or

b) converting 3-amino-5-[2-[(2-guanidino-4-thiazolyl)-methylthio]ethylamino]-4-methyl-1,2,4,6-thiatriazine 1,1-dioxide into a salt thereof, and isolating the resulting crystalline salt from the reaction mixture; or

c) converting a salt of 3-amino-5-[2-[(2-guanidino-4-thiazolyl)methylthio]ethylamino]-4-methyl-1,2,4,6-thiatriazine 1,1-dioxide into the free base, and isolating the resulting crystalline free base from the reaction mixture.

In all cases, a tautomer of a compound of formula A, C, D or F may be used.

In processes a), a leaving group may be substituted or unsubstituted, for example, a leaving group may be a lower alkoxy or phenylthio group, or an unsubstituted or substituted, for example, alkyl- or nitro-substituted phenyloxy or phenylthio group, and preferably represents a chlorine atom, or a methylthio, methylsulphinyl, methylsuphonyl, phenyloxy, tolyloxy or dimethylamino group.

Compounds A and B are generally reacted in a solvent or diluent, preferably an alcohol, acetonitrile, dimethylformamide, or dimethyl sulphoxide, at a temperature within the range of from, for example, 0 to 100°C, generally from 0 to 60°C. The compound of formula B should be reacted in the form of the free base, as shown. If it is initially present in the form of an acid addition salt, for example, as the hydrochloride or

hydrobromide, this should be converted into the free base during or, preferably, before reaction with compound A. Conversion is carried out with a base, for example, triethylamine, sodium hydroxide, potassium hydroxide, sodium ethoxide or potassium hydroxide.

Compound C is reacted with compound B under conditions similar to those described above, except that generally lower reaction temperatures are preferred in order that the two leaving groups $L^3$ and $L^4$ may be displaced selectively.

A compound of formula D is generally reacted with ammonia in a solvent, preferably a lower alcohol, especially methanol, ethanol or propanol, acetonitrile, or a mixture of two or more such solvents. The reaction may be carried out at a temperature within the range of from 10 to 50°C, preferably at room temperature.

The term "lower" is used herein to denote a group or molecule having from 1 to 4 carbon atoms.

The free base 3-amino-5-[2-[(2-guanidino-4-thiazolyl)-methylthio]ethylamino]-4-methyl-1,2,4,6-thiatriazine 1,1-dioxide crystallises readily from the reaction mixture , with cooling, if necessary, and the crystalline product can be isolated from the reaction mixture by any of the methods known for isolation of crystals from a reaction mixture, for example, by filtration or centrifugation.

Recrystallisation of the product may be carried out in known manner from a suitable solvent, for example, as described below.

A crystalline salt may be prepared according to process (b) from the free base 3-amino-5-[2-[(2-guanidino-4-thiazolyl)-methylthio]ethylamino]-4-methyl-1,2,4,6-thiatriazine 1,1-dioxide, which is preferably in crystalline form. The free base is reacted with the appropriate acid to give the desired salt. The conditions for formation of a crystalline salt may be determined empirically, for example, a solution of the free base in a

solvent is mixed with the appropriate acid in a solvent or diluent at a temperature within the range of from 20 to 100°C. The resulting mixture is then cooled, if appropriate, and allowed to stand at 0 to 30°C. until crystallisation takes place. Separation of the crystals from the mixture may be carried out by any of the known methods, for example, by filtration or centrifugation and, if desired, the product may be recrystallised from a suitable solvent, for example as described above.

In process (c), the crystalline free base 3-amino-5-[2-[(2-guanidino-4-thiazolyl)-methylthio]ethylamino]-4-methyl-1,2,4,6-thiatriazine 1,1-dioxide may be obtained by treatment of a salt thereof, preferably in crystalline form, with a base, preferably a slight excess thereof. Any salt may be used, and the base may be organic or inorganic, especially sodium or potassium hydrogen carbonate or sodium or potassium carbonate. The reaction may be carried out in a solvent and at room temperature. The free base crystallises readily from the resulting solution, and may be separated by any of the usual techniques, for example, by filtration or centrifugation. The product may be recrystallised, if desired.

Solvents for dissolution and recrystallisation for all of the processes described above may be selected, for example, from water, alcohols, especially lower alcohols, ketones, especially acetone, and mixtures of any two or more of these solvents. Other solvents may be used. Water and ethanol are particularly preferred.

As indicated above, in each case, a worker skilled in the art can determine the preferred reaction conditions, for example, regarding solvents and temperatures.

In many cases it is advantageous to induce crystallisation by means of seeding with the appropriate crystals.

As indicated above, the free base and its salts may crystallise in hydrated and/or solvated form, the nature and degree of which depend on the solvent or solvent

mixture used, and on the reaction conditions. Accordingly, all hydrated and/or solvated cystalline and non-crystalline forms of 3-amino-5-[2-[(2-guanidino-4-thiazolyl)-methylthio]ethylamino]-4-methyl-1,2,4,6-thiatriazine 1,1-dioxide are part of the present invention.

All tautomeric forms of 3-amino-5-[2-[(2-guanidino-4-thiazolyl)-methylthio]ethylamino]-4-methyl-1,2,4,6-thiatriazine 1,1-dioxide are part of the present invention, as are all tautomeric forms of each salt of the invention. The tautomers may be in crystalline or non-crystalline form.

As indicated above, 3-amino-5-[2-[(2-guanidino-4-thiazolyl)-methylthio]ethylamino]-4-methyl-1,2,4,6-thiatriazine 1,1-dioxide has histamine H-2 antagonist activity, and accordingly the free base or a salt thereof, especially in crystalline form, may be administered to a mammal at a dose of from 0.1 to 10mg per kg body weight per day, in a single dose or in divided doses, to treat those conditions resulting from stimulation by histamine of histamine H-2 receptors, either alone, for example, in inhibiting gastric acid secretion and thus treating its sequelae, for example, gastric and peptic ulcers; or together with a histamine H-1 antagonist, for example, in treating allergic and certain inflammatory conditions.

The crystalline forms of 3-amino-5-[2-[(2-guanidino-4-thiazolyl)-methylthio]ethylamino]-4-methyl-1,2,4,6-thiatriazine 1,1-dioxide and of salts thereof are useful in the manufacture and purification of the free base and its salts, particularly in large scale operations, and also in the formulation of pharmaceutical preparations.

The present invention provides a pharmaceutical preparation which comprises 3-amino-5-[2-[(2-guanidino-4-thiazolyl)-methylthio]ethylamino]-4-methyl-1,2,4,6-thiatriazine 1,1-dioxide or a physiologically tolerable salt thereof, in admixture or conjunction with a pharmaceutically suitable carrier.

The present invention also provides a pharmaceutical preparation which comprises 3-amino-5-[2-[(2-guanidino-4-thiazolyl)-methylthio]ethylamino]-4-methyl-1,2,4,6-thiatriazine 1,1-dioxide or a physiologically tolerable salt thereof in crystalline form, in admixture or conjunction with a pharmaceutically suitable carrier.

The salts used, and their crystalline form, are especially those characterised above or in the following Examples.

A pharmaceutical preparation of the invention may be suitable for use for enteral or parenteral administration, for example, as tablets, capsules, powders, syrups, suspensions, solutions, and preparations suitable for administration by injection or infusion.

A pharmaceutical preparation of the invention may also comprise one or more further pharmaceutically active substances, especially histamine H-1 antagonists.

A preparation of the invention may be in unit dosage form.

The present invention also provides the use of 3-amino-5-[2-[(2-guanidino-4-thiazolyl)-methylthio]-ethylamino]-4-methyl-1,2,4,6-thiatriazine 1,1-dioxide or a physiologically tolerable salt thereof, especially in crystalline form, in the manufacture of a medicament for the treatment of those conditions resulting from stimulation by histamine of histamine H-2 receptors, either alone, for example, in inhibiting gastric acid secretion and thus treating its sequelae, for example, gastric and peptic ulcers; or together with a histamine H-1 antagonist, for example, in treating allergic and certain inflammatory conditions.

The free base or a salt thereof may be administered at a dose of from 0.1 to 10 mg per kg body weight per day, in a single dose or in divided doses.

3-Amino-5-[2-[(2-guanidino-4-thiazolyl)-methylthio]-ethylamino]-4-methyl-1,2,4,6-thiatriazine 1,1-dioxide falls within the general formula I of our British Patent

Application No. 2 129 426A, which also describes a process for the production of a compound of formula I. Compounds A to G above fall within the general formulae II to VIII respectively of our Application No. 2 129 426A, which also describes the production of compounds II, IV and VI.

The following passage is quote, verbatim, from our British Application No. 83.25762:

" The present invention provides compounds of the general formula I, which are histamine H-2 antagonists:

$A - (CH_2)_n - X - (CH_2)_m - NH -$ 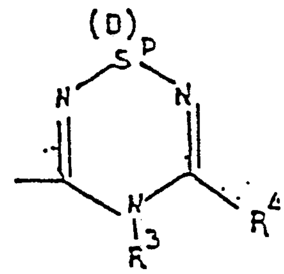 (I)

in which formula

A represents a phenyl, imidazolyl, thiazolyl, furyl, thienyl, or pyridyl radical; which radical may contain one or two substitutents, the first being selected from lower alkyl groups, and the second from lower alkyl, guanidino, and $-CH_2NR^1R^2$ groups, $R^1$ and $R^2$, which may be the same or different, each representing a hydrogen atom or a $(C_1-C_6)$alkyl group, or together with the nitrogen atom to which they are attached, may form a pyrrolidine, piperidine, morpholine, or N-methylpiperazine ring;

X represents -O-, -S-, or $-CH_2$;

n represents 0 or 1;

m represents 2 or 3;

p represents 1 or 2;

$R^3$ represents a hydrogen atom or a $(C_1-C_6)$alkyl, $(C_2-C_6)$-alkenyl, $(C_3-C_6)$alkynyl, phenyl, phenyl(lower)alkyl, carboxylic acyl$(C_1-C_6)$, phenyl(lower)acyl, nitrile, or $-N(alkyl)_2$ group;

$R^4$ represents a hydrogen atom, a $(C_1-C_6)$alkyl, $(C_2-C_6)$-alkenyl, $(C_3-C_6)$alkynyl, phenyl or phenyl(lower)alkyl group, an -O(lower)alkyl or -Oaryl group, a nitrile group, or an $-NR^5R^6$ group, in which $R^5$ and $R^6$, which may be the same or different, each represents a hydrogen atom, a $(C_1-C_6)$alkyl, $(C_2-C_6)$-alkenyl, $(C_3-C_6)$alkynyl, $(C_3-C_7)$cycloalkyl, phenyl or phenyl(lower)alkyl group, a $-(CH_2)_m-X-(CH_2)_n-A$ group, or an $-NH_2$ or nitrile group, (m, n, X and A being as defined above), or together with the nitrogen atom to which they are attached may form a 5 or 6 membered ring optionally containing a second nitrogen atom or an oxygen atom, or

$R^3$ and $R^4$ together with the atoms to which they are attached may form a 5 or 6 membered ring, containing one or more further heteroatoms selected from oxygen, nitrogen and sulphur atoms.

This invention also provides salts of a compound of formula I, especially physiologically tolerable salts.

Alkyl, alkenyl, alkynyl, and acyl groups in the present specification may be branched or unbranched and may be unsubstituted or substituted, for example, by one or more groups selected from hydroxyl groups; $-OR^7$ groups in which $R^7$ represents a $(C_1-C_6)$alkyl or a phenyl group; $(C_3-C_7)$cycloalkyl groups; $-NR^8R^9$ groups in which $R^8$ and $R^9$, which may be the same or different, each represents a hydrogen atom, a lower alkyl group, an aryl group or a lower acyl group, and may together with the nitrogen atom to which they are attached form a 5 or 6 membered ring; $-COOR^{10}$ groups in which $R^{10}$ represents a hydrogen atom, an alkali metal atom, for example, Na or K, or a lower alkyl group; $-CONR^8R^9$ in which $R^8$ and $R^9$ are as defined above; lower alkylsulphonyl and arylsulphonyl groups; cyano groups; and phenyl groups which may be substituted by one or two substituents, which may be the same or different selected from a lower alkyl, lower alkoxy, methylenedioxy, phenoxy, halogen, dimethylaminomethyl, trifluoromethyl, nitro, cyano, sulphonic acid, sulphonamide, amino, mono- lower alkyl-amino, di-lower alkyl amino groups; aromatic and non-aromatic heterocyclic groups having 5 to 8 ring members and one or two hetero atoms selected from oxygen, sulphur and nitrogen, and optionally having a lower alkyl substituent on a ring nitrogen atom, for example, furyl, tetrahydrofuryl, thienyl, pyridyl, dihydropyranyl, pyrrolidinyl, N-lower alkyl-pyrrolidinyl and piperidyl groups.

A phenyl group other than a phenyl group A (which is defined above) may be unsubstituted or substituted as defined immediately above for phenyl substituents of aliphatic and acyl groups.

In the present specification the term "lower" is used to denote a group having up to 4 carbon atoms. The term "halogen" denotes chlorine, bromine, iodine and fluorine. The term "aryl", unless otherwise described, denotes aromatics having 6 to 12 carbon atoms, such as phenyl, naphthyl, bisphenylyl, particularly phenyl;

aralkyl means aralkyls having 7 to 13 C atoms, such as benzyl, phenethyl.

It will be appreciated by a worker skilled in the art that a combination of substituents that are incompatible for steric reasons or because of potential inter-reactions should not be chosen. The worker will also use his normal discretion in the number of substituents chosen.

The present invention also provides a process for the production of a compound of the general formula I, which comprises

a) reacting a 1,2,4,6-thiatriazine of the general formula II

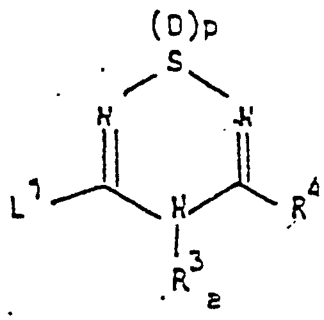

II

in which $L^1$ represents a halogen atom, an alkoxy or aryloxy group, an alkylthio or arylthio group, or an alkylsulphonyl or arylsulphonyl group, p and $R^4$ are as defined above, and $R^3_a$ is as defined above for $R^3$ except that $R^3_a$ may not represent a hydrogen atom,

with a compound of the general formula III

$$A-(CH_2)_n-X-(CH_2)_m-NH_2 \qquad III$$

in which A, X, m and n are as defined for formula I, or

b) reacting a 1,2,4,6-thiatriazine of the general formula IV

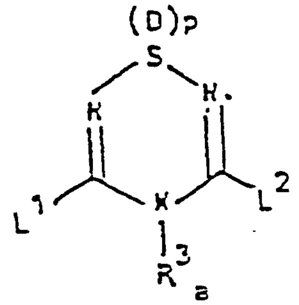

IV

in which $L^1$ and $L^2$, which may be the same or different, each represents a halogen atom, an alkoxy or aryloxy group, an alkylthio or arylthio group, or an alkylsulphonyl or arylsulphonyl group, and p and $R^3_a$ are as defined above, with a compound of formula III as defined above, and reacting the resulting compound of formula V

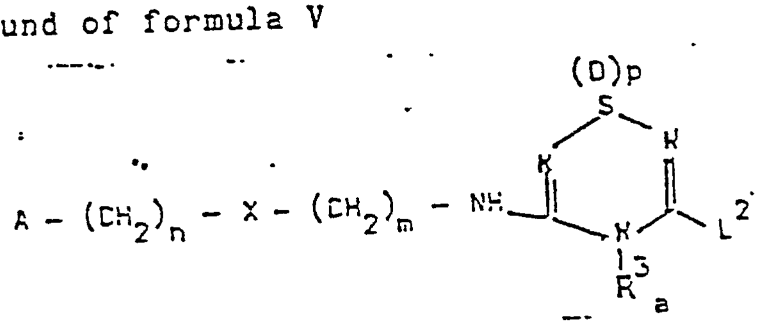

V

with an amino compound $HNR^5_aR^6_a$ in which $R^5_a$ and $R^6_a$ are as defined for $R^5$ and $R^6$ in formula I and, in addition, either or both of $R^5_a$ and $R^6_a$ may represent a trialkylsilyl group, for example, a $-(CH_3)_3Si$ group; or

c) reacting a 1,2,4,6-thiatriazine of the general formula II as defined above with an amino thiol of formula VI

$HX_a - (CH_2)_m - NH_2$ VI

in which $X_a$ is -O- or -S-, and m is as defined for formula I, and reacting the resulting a compound of formula VII

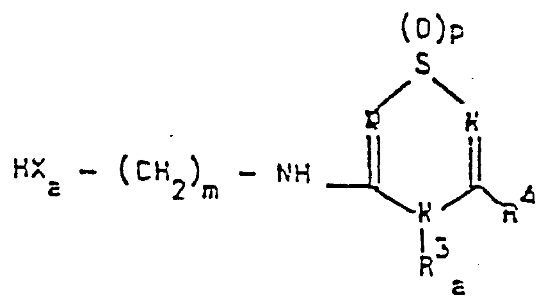

VII

in which $R^3_a$, $R^4$, p and m are as defined above, with a compound of the general formula VIII .

$A - (CH_2)_{n^1} - L^3$ VIII

in which A is as defined for formula I, $n^1$ represents 1, and $L^3$ is a suitable leaving group, for example, a halogen atom, a hydroxy group, an alkoxy group, or a sulphonate ester, and, if desired, carrying out any one or more of the following reactings in any desired order:-

(i) converting a group $R^3_a$ into a hydrogen atom,

(ii) converting a group $R^3$ and/or a group $R^4$ into another group $R^3$ and/or $R^4$, respectively,

(iii) converting an acid addition salt of formula I into the corresponding free base or converting a free base into an acid addition salt,

(iv) oxidising a compound or formula I in which p represents 1 to give the corresponding compound in which p represents 2.

In addition to serving as a substituent in the final product, $R^3$ may also act as a protecting group in the above chemical transformations and may subsequently be removed, for example, by hydrogenolysis or by acid or base catalysed hydrolysis to provide a compound of the general formula I in which the thiatriazine ring carries a hydrogen substituent. Protecting groups which come within the definition of

$R^3$ given in formula I are t-butyl, lower alkyl carbonate, benzyl and benzoyl groups, for example.

Oxidation may be carried out by a know method, for example, as described below. Interconversions of substitutents may also be carried out by known methods, for example, the hydrolysis of a cyano group to an acid or amide.

Compounds II and III are generally reacted in a solvent or diluent, preferably an alcohol, DMF, or DMSO, at a temperature within the range of from, for example, from 0 to 100°C, generally from 0 to 60°C. The compound of formula III should be reacted in the form of the free base, as shown. If it is initially present in the form of an acid addition salt, for example, as the hydrochloride or hydrobromide, this should be converted into the free base during or, preferably, after reaction with compound II. Conversion is carried out with a base, for example, triethylamine, sodium hydroxide or potassium hydroxide.

Compound IV is reacted with compound III under conditions similar to those described above, except that generally lower reaction temperatures are preferred in order that the leaving groups $L^1$ and $L^2$ may be displaced selectively.

The reaction of compound V with an amino compound $HNR^5_aR^6_a$ is generally carried out in an alcoholic solvent at a temperature from 0 to 60°C. The reaction in all cases is faster when p= 2 than when p= 1.

A compound of formula I may be converted into its salt form in the usual manner by reaction with an acid. Examples of physiologically tolerable acid addition salts are those with hydrobromic, hydrochloric, sulphuric, acetic, malonic, maleic, fumaric, succinic, citric, tartaric and isethionic acids.

A compound of formula I in the form of an acid addition salt may be converted into the free base form by reaction with a base in the usual manner.

A number of compounds of the type represented by the general formula III are known, see, for example, GB Specification 2,001,624 A; U.S. Patent 3,950,333; U.S. Patent 4,128,658; and Belgian Patents 867,106 and 875,846.

A few examples of the required thiatriazine intermediates represented by the general formulae II, IV and VI are disclosed in the chemical literature, see for example, German specification DE 2,026,625 and DE 2,943,703; U.S. patent 4,013,447; Heterocycles 12, 1199 (1979); and Chem. Ber. 109 2107 (1976).

However, many of the specific intermediates required for the present invention have not previously been described and form part of the present invention.

Some thiatriazines of the general formula II may be prepared as shown in Scheme I.

Scheme I

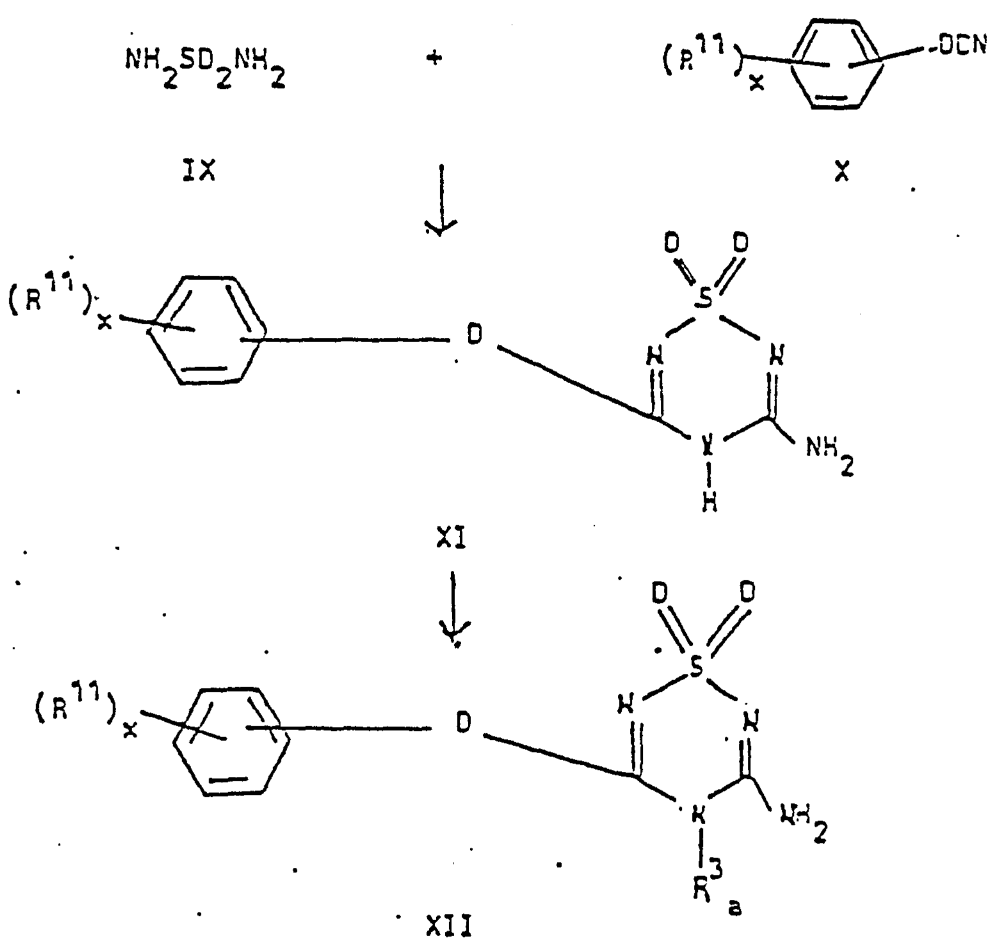

IX

X

XI

XII

$R^{11}$ represents an alkyl, dialkylamino, acylamino, nitro, halogen, aryl, nitrile, alkoxy, aryloxy or acyloxy group, and x represents 1 or 2. Preferably one group $R^{11}$ is present, ortho or, preferably, para to the -OCN group.

Sulphamide IX may be reacted with an aryl cyanate of formula X in the presence of a base, for example, $Na_2CO_3$, according to the method described in DE 2,026,625 to give a thiatriazine of formula XI. This compound may be alkylated on the nitrogen atom in the 4-position using an alkylating agent, for example, an alkyl halide or alkyl sulphate, for example, methyl iodide, benzyl bromide or dimethyl sulphate.

The alkylation of the 4-amino group may be carried out in a known manner, for example, in the presence of a base, for example, sodium methoxide or di-isopropylethylamine, in a solvent, for example, methanol or acetonitrile, and generally at a temperature within the range of 20 to 100°C.

Certain thiatriazines of the general formula IV may be prepared as shown in Scheme II or Scheme III.

Scheme II

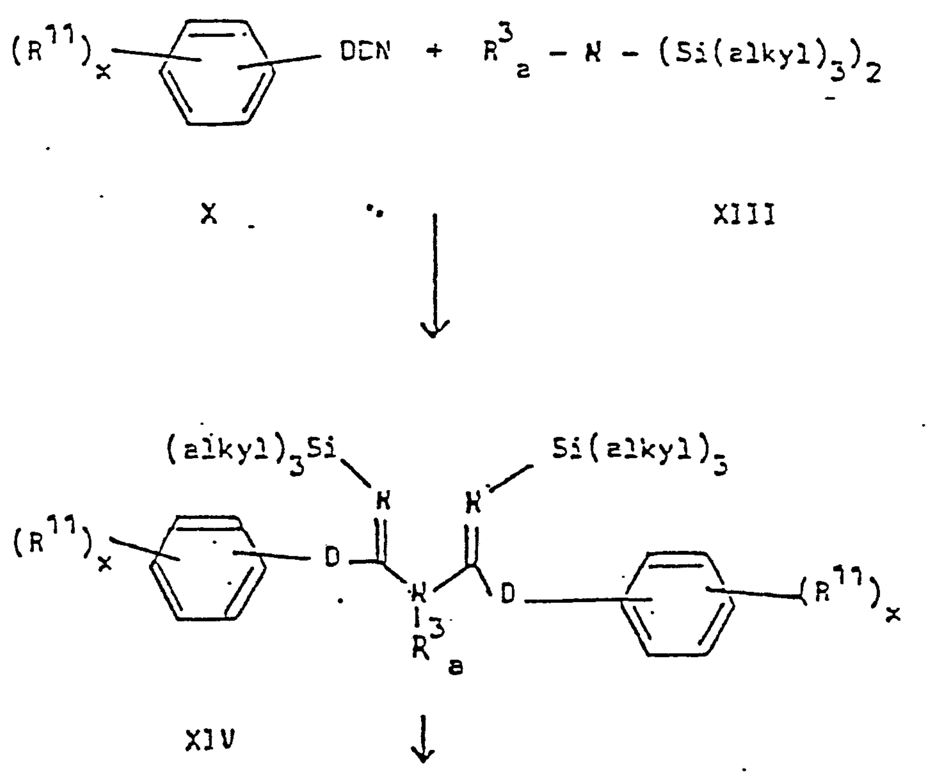

X

XIII

XIV

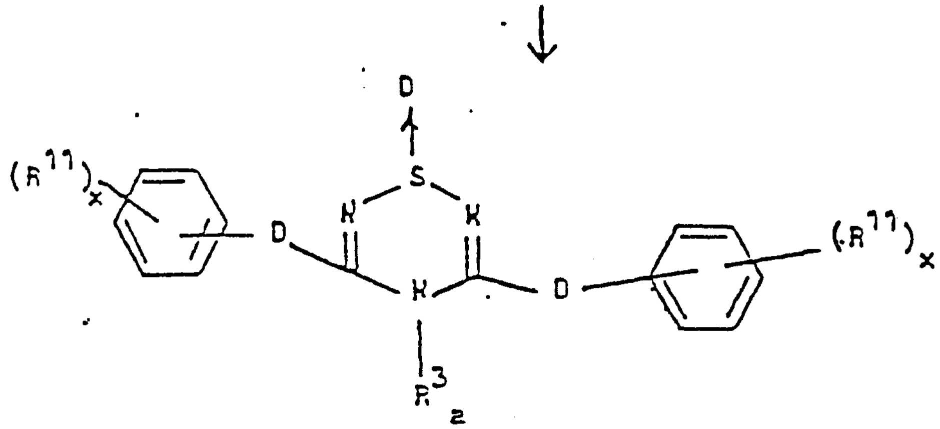

XV

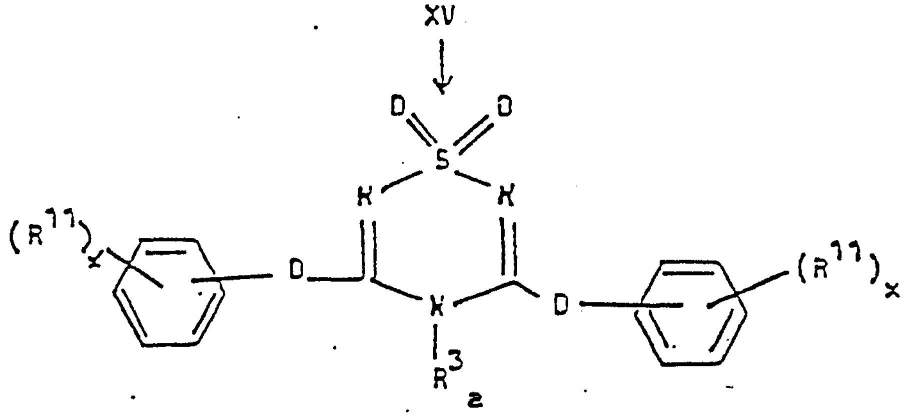

XVI

In Scheme II, an aryl cyanate of formula X in which $R^{11}$ and x are as defined above is reacted with a substituted bis-trialkylsilylamine of formula XIII in which $R^3{}_a$ is as defined above to give a compound of formula XIV. The trialkyl group is preferably a trimethyl group. This reaction may be carried out as described in Chem. Ber. 101 3185 (1968). Cyclisation of compound XIV to give the thiatriazine of formula XV may be carried out by reaction of compound XIV with thionyl chloride, thionylaniline or thionyldiimidazole. A compound XV may be used diretly in a condensation reaction with a compound of formula III to give a compound of formula I, or may first be oxidised to the corresponding 1,1-dioxide XVI, using an oxidising agent, for example, a peracid, for example, m-chloroperbenzoic acid, hydrogen peroxide, an alkyl hydroperoxide, for example, t-butyl hydroperoxide, or a permanganate, for example potassium permanganate.

Scheme III

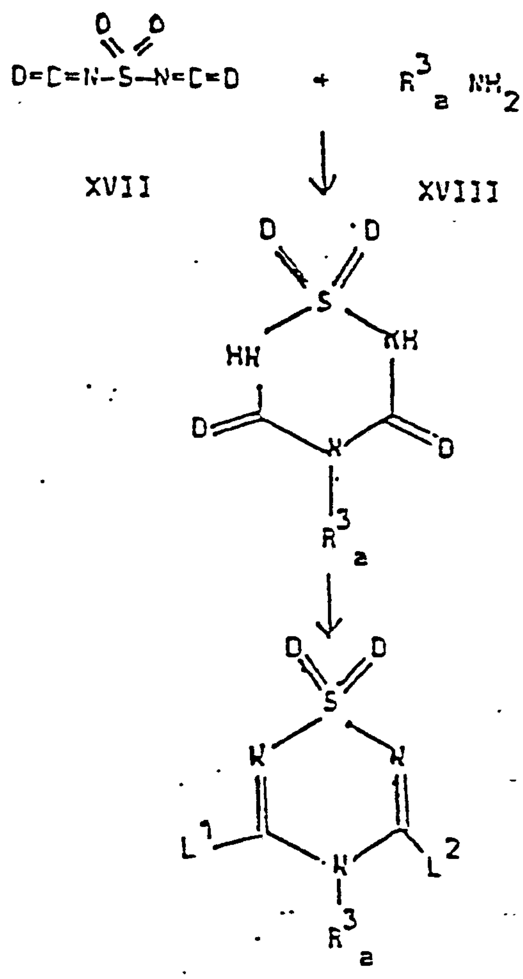

XVII

XVIII

XIX

XX

Sulphonyl di-isocyanate XVII may be reacted with a primary amine of formula XVIII in which $R^3_a$ is as defined above according to DE-OS 2,337,867, to provide the thiatriazine of formula XIX. Conversion of this compound to the thiatriazine XX having leaving groups $L^1$ and $L^2$ at the 3 and 5 positions (ie a compound of formula IV in which p= 2) may be accomplished by a variety of methods, for example, by treatment with $PCl_5$ or $COCl_2$/DMF in a solvent, for example, $POCl_3$, $CCl_4$ or $ClCH_2CH_2Cl$, to give $L^1 = L^2 = Cl$, or by treatment with phosphorus pentasulphide or p-methoxyphenylthionophosphine sulphide dimer with subsequent alkylation to give $L^1 = L^2$ = alkylthio.

It will be appreciated by those skilled in the art that it is possible to convert a thiatriazine intermediate of formula IV into a thiatriazine intermediate of formula II by, for example, reaction with a primary or secondary amine $NHR^5{}_aR^6{}_a$.

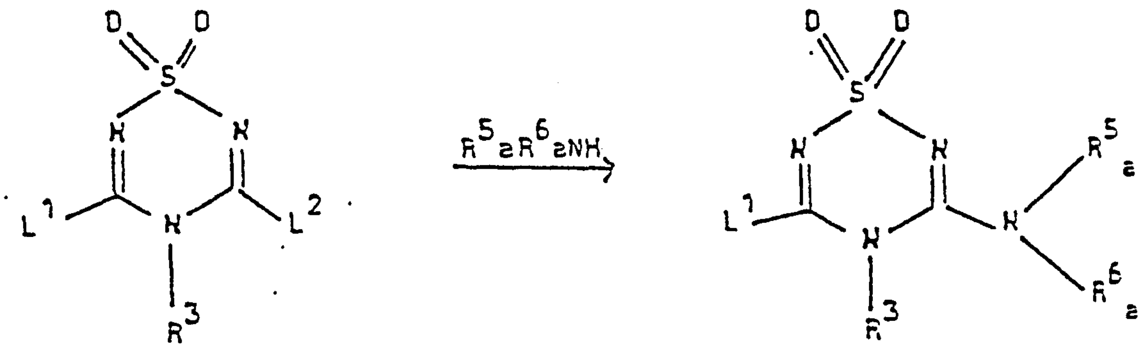


IV

II

Certain thiatriazines of general formula II may be prepared as shown in Scheme IV.

Scheme IV



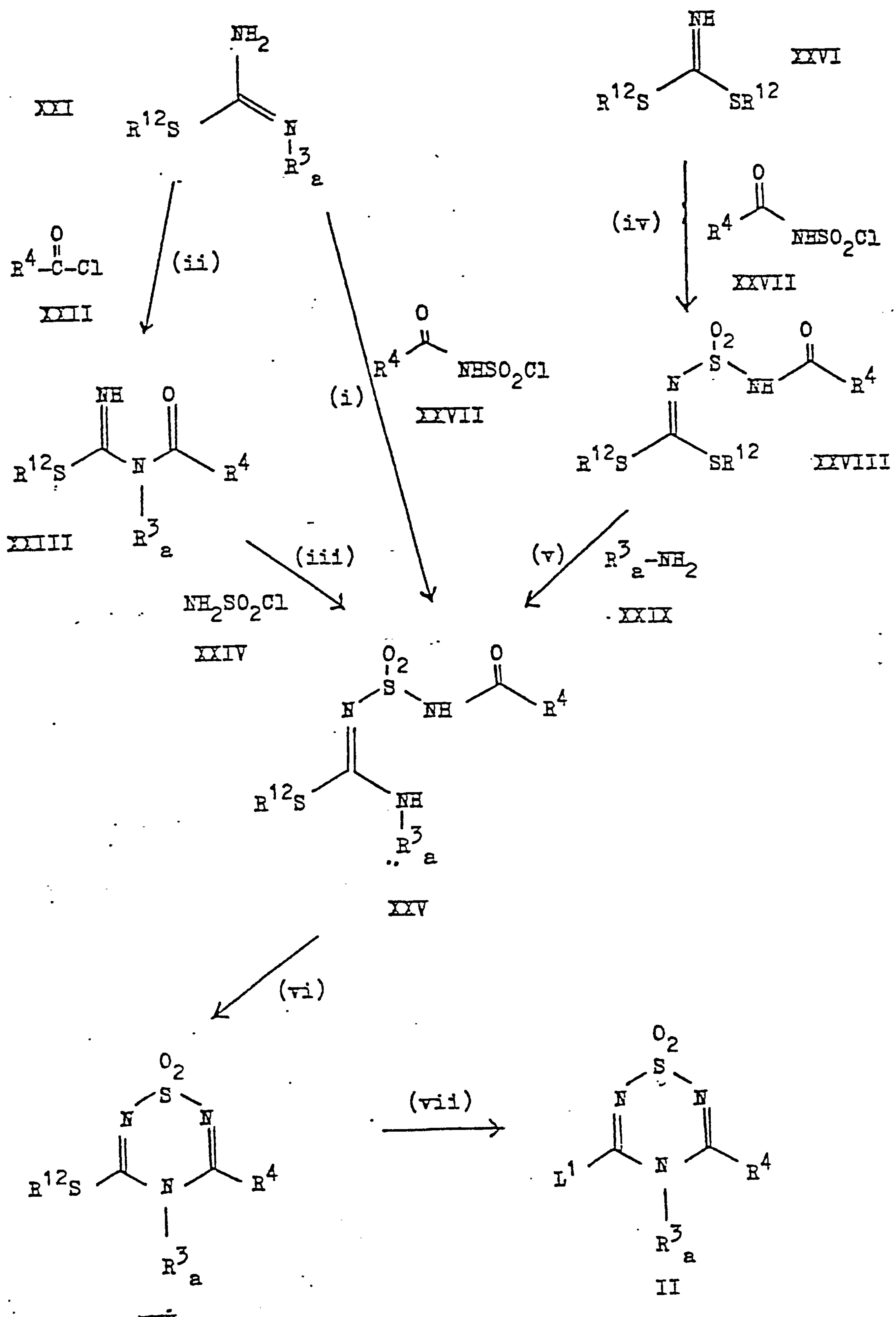
XXI
XXVI
(ii)
XXII
(i)
XXVII
(iv)
XXVII
XXIII
XXVIII
(iii)
XXIV
(v)
XXIX
XXV
(vi)
(vii)
II

In the above reaction scheme, $R^3_a$, $R^4$ and $L^1$ are as defined above, and $R^{12}$ represents an alkyl group, for example an alkyl group having up to 4 carbon atoms, especially a methyl group; an aryl group, especially a phenyl group; or an aralkyl group, especially a benzyl group. In compound XXVI, the two groups $R^{12}$ may be the same or different. In compound XXI, $R^{12}$ is for example, a methyl group, and in compound XXVI, for example, one of the two groups $R^{12}$ is a methyl group and the other is a phenyl group, or both are methyl groups, or both are phenyl groups.

Acylaminosulphonylchlorides of formula XXVII are known, see for example, R. Graf, German Specifications 931,225 and 931,467. Dithioiminocarbonates of formula XXVI are also known, see for example, Z. Chem. 8, 459 - 460 (1968). (The term "known" is used herein to mean in actual use in the art or described in the literature of the art.)

An N-substituted isothiourea XXI may be reacted with an acylaminosulphonyl chloride XXVII in the presence of a base, for example, triethylamine or diisopropylethylamine, in an aprotic solvent, for example, dichloromethane, tetrahydrofuran or acetonitrile, generally at a temperature within the range of from -20 to +30°C, to give the key intermediate of formula XXV (reaction i).

A compound of formula XXV may also be prepared from an isothiourea XXI by another route (reactions ii and iii).

First, the isothiourea XXI reacted with a carboxylic acid chloride XXII, in the presence of a base, for example, triethylamine or diisopropylethylamine, in an aprotic solvent, for example, dichloromethane, tetrahydrofuran or acetonitrile, generally at temperature within the range of from -50 to 0°C, to give a mixture of isomers from which the predominant one, compound XXIII, can be separated by conventional means, for example, by fractional crystallisation or by chromatography. Compound XXIII is then reacted with aminosulphonyl chloride XXIV under conditions analogous to those described above for reaction (i). Migration of the acyl group results in the formation of compound XXV.

Compound XXV may be prepared by a third method (reactions iv and v), which comprises reacting a dithioiminocarbonate XXVI with an acylaminosulphonyl chloride XXVII under conditions analogous to those described above for reaction (i) and then displacing an $-SR^{12}$ group from the resulting compound XXVIII with primary amine of formula XXIX.

Heating a compound of formula XXV in an aprotic solvent, for example, tetrahydrofuran, dioxane, toluene, xylene or ethylene glycol dimethyl ether, generally at a temperature within the range of from 80 to 160°C, affords a thiatriazine of formula XXX.

In some compounds of formula XXX, the group $R^{12}S-$ is a suitable leaving group $L^1$, ie such compounds fall within the general formula II, but in other compounds of formula XXX it is necessary or preferable to convert a goup $R^{12}S-$ into a leaving group $L^1$ that is more readily displaced by a nucleophile. Such a conversion may be carried out by known methods, for example, by oxidising the sulphide group $R^{12}S-$ to the corresponding sulphoxide group using, for example, a peracid, for example, m-chloroperbenzoic acid, hydrogen peroxide, or a periodate, perborate or permanganate salt; or by replacing the sulphide group $R^{12}S-$ by a halogen atom, for example, by treating compound XXX with excess chlorine in an inert solvent, for example, chloroform, tetrahydrofuran or ethyl acetate, generally at a temperature within the range of from 20 to 100°C, and preferably in the presence of a catalyst, for example, zinc chloride.

It will be appreciated by those skilled in the art that compounds of the general formula I may exist as one or more tautomeric isomers, especially those structures where $R^3$ represents a hydrogen atom and $R^4$ represents a group $-NR^5R^6$ in which $R^5$ or $R^6$ represents a hydrogen atom, for example, as shown below:

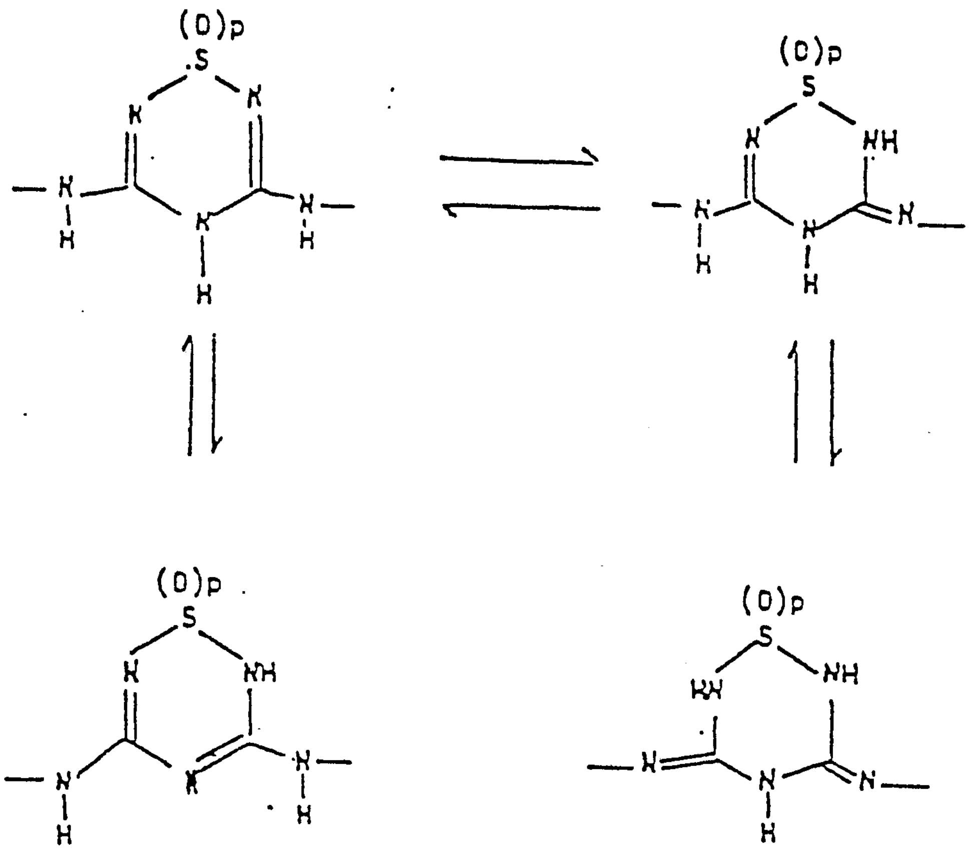

This tautomerism may also occur when the thiatriazine ring is substituted by carbon substituents:

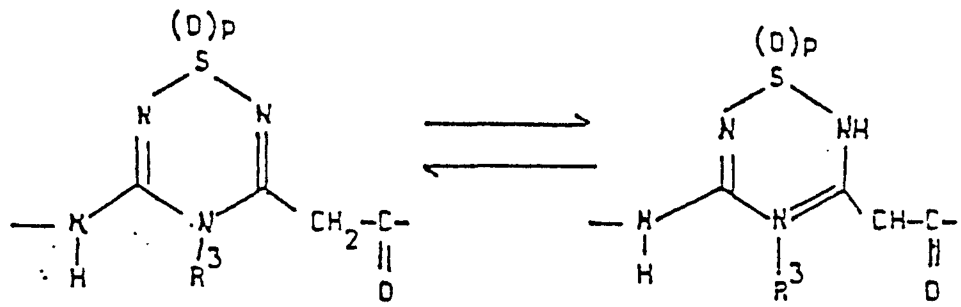

All possible tautomeric forms of formula I are part of the present invention.

It will also be appreciated that when p represents 1, the sulphur-oxygen bond in formula I can exist in two stereoisomeric configurations: R and S. All stereoisomers of formula I are also part of the present invention.

Any reference to a compound of formula I herein includes all possible tautomeric and stereoisomeric forms of that compound. Moreover, all possible tautomeric and stereoisomeric forms of any other compound mentioned in the present specification are similarly included within the general references to that compound" (End of quote from 83.25762).

The following Examples are given by way of illustration only, and are not limiting.

In the Examples, melting points were measured with a Reichert hot-stage apparatus. All temperatures are expressed in degrees Celcius. Unless specified otherwise, infra red (IR) spectra were measured as KBr discs, and in each of Figures 1 to 4 of the accompanying drawings, which show various IR spectra, the ordinate is the transmittance in % and the abscissa is the wave number in $cm^{-1}$. Unless specified otherwise, the N.M.R. (250 MHz) spectra were measured in dimethylsulphoxide-$d_6$ using tetramethylsilane as internal standard. The X-ray powder diffraction patterns were determined with a Philips horizontal goniometer PW 1380 driven by a PW 1710 diffracter controller and using a PM 8210 printer/recorder. The speed of the goniometer was 1.0 and the lower window level set at 35 and the upper level set at 65. Samples were mounted on double sided adhesive tape and exposed to Ni filtered Cu radiation. The X-ray wavelength used for the calculations was 1.54179 Å.

In the description above and in the Examples below, X-ray powder diffraction patterns are described in terms of 'd' spacings and relative intensities (I) using the following abbreviations: s=strong, m=medium, w=weak, v=very.

It will be appreciated by one skilled in the art that the melting point of a product will depend on the degree of hydration thereof.

EXAMPLE 1

3-amino-5-[2-[(2-guanidino-4-thiazolyl)methylthio]-ethylamino]-4-methyl-1,2,4,6-thiatriazine 1,1-dioxide

(A1) 20 ml of an ethanolic ammonia solution (saturated), was added to 1.0 g of a stirred supension of 5-[2-[(2-guanidino-4-thiazolyl)methylthio]ethylamino]-4-methyl-1,2,4,6-thiatriazine 1,1-dioxide in 20 ml of dry ethanol, and the mixture was stirred at room temperature overnight. The product was filtered off, washed with cold ethanol, and dried *in vacuo* over $P_2O_5$. The title compound was obtained as white crystals (0.75 g), m.p. 213-215°C. $\nu_{max}$ (Nujol mull) 3460, 3420, 3350, 3105, 3170, 3215, 1690, 1640, 1610, 1565, 1545, 1405, 1340, 1260, 1215, 1160, 1120, 1070, 1050, 1010, 990, 970, 875, 840 and 720 $cm^{-1}$.

N.M.R. : 2.62 (2H, t), 3.19 (3H, s), 3.35 (2H, t), 3.61 (2H, s), 6.57 (1H, s), 6.84 (4H, broad s, exchangeable), 7.34 (2H, broad s, exchangeable) and 7.63 (1H, t, exchangeable). The spectrum also contains ethanol.

(A2)Recrystallisation of the product from water afforded a crystalline solid with identical physical properties to those described in (B2) below.

(B1) 2.0 g of sodium hydrogen carbonate in 20 ml of water was added to a hot solution of 3.68 g of 3-amino-5-[2-[(2-guanidino-4-thiazolyl)methylthio]ethylamino]-4-methyl-1,2,4,6-thiatriazine 1,1-dioxide hemisuccinate in 120 ml of water. The solution was allowed to stand at room temperature overnight. The product was filtered off, washed with a small amount of cold water and dried *in vacuo* over $P_2O_5$. The resulting dihydrate of the free base was obtained as colourless needles (3.04 g), m.p. 139-145°C.

Microanalysis: Found: C, 28.09; H, 4.85; N, 29.43.

$C_{10}H_{17}N_9O_2S_3.2H_2O$ requires C, 28.09; H, 4.95; N, 29.49%.

The IR spectrum (KBr disc) is shown in Figure 1 of the accompanying drawings. The characteristic bands are as follows:
$\nu_{max}$ 3480, 3380, 3600-2500 (very broad), 1640 (very broad), 1615, 1580, 1545, 1520, 1500, 1455, 1410, 1330, 1260, 1220, 1190, 1160, 1130, 1085, 1060, 1000, 850, 710 and 670 $cm^{-1}$.

(B2) Recrystallisation of the dihydrate from water gave the monohydrate as colourless needles, m.p. 148-151°C.

Microanalysis: Found: C, 29.44; H, 4.63; N,30.77.
C H N O S.1H O requires C, 29.33; H, 4.68; N, 30.78%.

The IR spectrum (KBr disc) is shown in Figure 2 of the accompanying drawings. The characteristic bands are as follows:
$\nu_{max}$ 3580, 3420, 3340, 3180, 1660, 1610, 1570, 1530, 1520, 1455, 1410, 1365, 1340, 1270, 1250, 1220, 1190, 1130, 1120, 1075, 1010, 990, 970, 870, 860, 730, 700 and 660 $cm^{-1}$.

X-Ray Diffraction Pattern (given as d(A), I for each value):
2.18 vw; 2.29 vw; 2.58 vw; 2.64 vw; 2.88 vw; 2.97 vw; 3.05 w; 3.17 w; 3.25 w; 3.33 w; 3.42 w; 3.50 w; 3.72 m; 4.07 m; 4.36 m; 5.10 m; 6.32 m; 6.74 s; 7.39 m.

NMR: 2.63 (2H, t), 3.19 (3H, s), 3.35 (2H, t), 3.62 (2H, s), 6.57 (1H, s), 6.83 (4H, very broad s, exchangeable), 7.33 (2H, broad s, exchangeable) and 7.61 (1H, t, exchangeable).

<u>EXAMPLE 2</u>

<u>Hydrochloride salt of 3-amino-5-[2-[(2-guanidino-</u>

4-thiazolyl)methylthio]ethylamino]-4-methyl-1,2,4,6-thiatriazine 1,1-dioxide

2 ml of hydrochloric acid was added to a suspension of 1.5 g of 3-amino-5-[2-[(2-guanidino-4-thiazolyl)-methylthio]ethylamino]-4-methyl-1,2,4,6-thiatriazine 1,1-dioxide in 20 ml of water and the mixture was warmed until the solid dissolved. The solution was then allowed to cool and stand at room temperature until crystallisation was complete. The product was filtered off, washed with a little cold water, and dried in vacuo over $P_2O_5$. The hydrochloride salt was obtained as buff coloured crystals (1.4 g), m.p. 243-245°C. (dec.)

Recrystallisation of the product from water gave colourless crystals, m.p.250-252°C. (dec.)

The IR spectrum (KBr disc) is shown in Figure 3 of the accompanying drawings. The characteristic bands are as follows:

$\nu_{max}$ 3450-2500 (very broad), 3390, 3280, 3180, 3100, 3060, 1695, 1665, 1610, 1560, 1525, 1485, 1460, 1435, 1410, 1335, 1260, 1215, 1190, 1150, 1130, 1085, 1050, 980, 860, 720 and 650 $cm^{-1}$.

X-Ray Diffraction Pattern given as d(Å), I for each value):

2.53 vw; 2.69 w; 2.83 w; 2.89 m; 2.94 m; 3.29 m; 3.52 m; 3.61 m; 3.92 s; 4.23 w; 4.38 s; 4.71 m; 5.24 m.

NMR: 2.61 (2H, t), 3.20 (3H, s), 3.34 (2H, t), 3.78 (2H, s), 7.24 (1H, s), 7.38 (2H, broad s, exchangeable), 7.75 (1H, t, exchangeable), 8.24 (4H, broad s, exchangeable) and 12.3 (1H, broad s, exchangeable).

Microanalysis: Found: C, 27.96; H, 4.16; N, 29.53.
$C_{10}H_{18}ClN_9O_2S_3$ requires C, 28.07,; H, 4.24; N, 29.46%.

EXAMPLE 3

Sulphate salt of 3-amino-5-[2-[(2-guanidino-4-thiazolyl)-methylthio]ethylamino]-4-methyl-1,2,4,6-thiatriazine 1,1-dioxide

1 ml of 2M sulphuric acid was added to a suspension of 0.5 g of 3-amino-5-[2-[(2-guanidino-4-thiazolyl)methylthio]ethylamino]-4-methyl-1,2,4,6-thiatriazine 1,1-dioxide in 10 ml of water and the reaction mixture was warmed until the solid disolved. The solution was cooled and the product filtered off, washed with a small amount of cold water and dried *in vacuo* over $P_2O_5$. The sulphate salt was obtained as fine needles (0.54 g), m. p. 212-215°C.

Recrystallisation of the product from water gave colourless needles, m.p. 219-220°C.

$\nu_{max}$ (KBr disc) 3200-2500 (very broad), 1705, 1660, 1620, 1570, 1530, 1460, 1440, 1410, 1340, 1280, 1230, 1215, 1190, 1130, 1070, 1010, 990, 860, 810 and 715 $cm^{-1}$.

NMR: 2.62 (2H, t), 3.19 (3H, s), 3.35 (2H, t), 3.69 (2H, s), 6.92 (1H, s), 7.36 (2H, broad s, exchangeable), 7.57 (4H, broad s, exchangeable) and 7.65 (1H, t, exchangeable).

Microanalysis: Found: C, 26.17; H, 4.25; N, 27.34. $C_{10}H_{17}N_9O_2S_3.\frac{1}{2}H_2SO_4.H_2O$ requires C, 26.19; H, 4.40; N, 27.49%.

EXAMPLE 4

Hemisuccinate salt of 3-amino-5-[2-[(2-guanidino-4-thiazolyl)methylthio]ethylamino]-4-methyl-1,2,4,6-thiatriazine 1,1-dioxide

A warm solution of 0.12 g of succinic acid in 5 ml of water was added to a hot solution of 0.39 g of 3-amino-5-[2-[(2-guanidino-4-thiazolyl)methylthio]-ethylamino]-4-methyl-1,2,4,6-thiatriazine 1,1-dioxide in 30 ml of water. The solution was allowed to cool and then

stand at room temperature overnight. The product was filtered off, washed with cold ethanol and dried *in vacuo* over $P_2O_5$.

Recrystallisation from water gave a microcrystalline solid (0.34 g, m.p. 137-140°C.

The IR spectrum (KBr disc) is shown in Figure 4 of the accompanying drawings. The characteristic bands are as follows:

$\nu_{max}$ 3600-2500 (very broad), 1705, 1655 (sh), 1640 (sh), 1620, 1560-1530 (broad), 1490, 1460, 1440, 1405, 1385, 1330, 1240, 1220, 1195, 1150, 1120, 1090, 1060, 985, 855, 805, 715 and 660 $cm^{-1}$.

X-Ray Diffraction Pattern (given as d(A), I for each value): 2.39 vw; 3.12 vw; 3.39 vw; 3.46 s; 3.62 m; 4.01 m; 4.11 m; 4.69 m; 5.26 w; 5.97 m; 6.76 m; 7.20 m; 7.99 m.

NMR: 2.41 ((2H, s), 2.63 (2H, t), 3.19 (3H, s), 3.36 (2H, q), 3.35 (approx. 2H, very broad s, exchangeable), 3.62 (2H, s), 6.59 (1H, s), 6.87 (4H broad s, exchangeable), 7.33 (2H, slightly broad s, exchangeable) and 7.61 (1H, t, exchangeable).

Microanalysis: Found: C, 29.68; H, 4.83; N, 26.26. $C_{12}H_{20}N_9O_4S_3 \cdot 2H_2O$ requires C, 29.62; H, 4.97; N, 25.91%.

EXAMPLE 5

Maleate salt of 3-amino-5-[2-[(2-guanidino-4-thiazolyl)-methylthio]ethylamino]-4-methyl-1,2,4,6-thiatriazine 1,1-dioxide

A solution of 0.35 g of maleic acid in 10 ml of ethanol was added to a hot solution of 1.17 g of 3-amino-5-[2-[(2-guanidino-4-thiazolyl)methylthio]ethylamino]-4-methyl-1,2,4,6-thiatriazine 1,1-dioxide in ethanol. The solution was allowed to stand at room

temperature until crystallisation took place. The solid was then filtered off, washed with cold ethanol and dried in vacuo over $P_2O_5$. The maleate was obtained as a white solid (1.33 g), andrecrystallistion of the product from water afforded fine crystals, m.p. 192-194°C.

$\nu_{max}$ (Nujol mull) 3330 (broad), 3180, 3110, 1700, 1660, 1635, 1610, 1560, 1535, 1400, 1340, 1270, 1235, 1215, 1130, 1120, 1075, 1005, 995, 890, 865, 835, 750 and 720 $cm^{-1}$

NMR: 2.62 (2H, t), 3.20 (3H, s), 3.35 (2H, m), 3.75 (2H, s), 6.06 (2H, s). 7.14 (1H, s), 7.38 (2H, broad s, exchangeable), 7.67 (1H, t, exchangeable) and 7.97 (4H, very broad s, exchangeable).

Microanalysis: Found: C, 33.04; H, 4.13; N, 24.61.
$C_{14}H_{21}N_9O_6S_3$ requires C, 33.13; H, 4.17; N,24.84%.

EXAMPLES 6 TO 9

The following salts were obtained from 3-amino-5-[2-[(2-guanidino-4-thiazolyl)methylthio]ethylamino]-4-methyl-1,2,4,6-thiatriazine 1,1-dioxide and the appropriate acid, using a procedure analogous to that described in Example 5:

EXAMPLE 6

Hemi-oxalate salt of 3-amino-5-[2-[(2-guanidino-4-thiazolyl)methylthio]ethylamino]-4-methyl-1,2,4,6-thiatriazine 1,1-dioxide

White crystals were obtained on recrystallisation from water, m.p. 166-170°C (dec)

$\nu_{max}$ (Nujol mull) 3400 (broad), 3200 (broad), 1710, 1660, 1620, 1560, 1540, 1420, 1345, 1305, 1270 (broad), 1235, 1215, 1130 (broad), 1070, 1050, 1000, 980, 870, 840,

765, 725 and 715 $cm^{-1}$.

NMR: 2.61 (2H, t), 3.20 (3H, s), 3.35 (2H, m), 3.69 (2H, s), 6.88 (1H, s), 7.38 (2H, broad s, exchangeable), 7.60 (4H, very broad s, exchangeable) and 7.68 (1H, t, exchangeable).

Microanalysis: Found: C, 28.60; H, 4.14; N, 27.14. $C_{11}H_{18}N_9O_4S_3.1.5H_2O$ requires C, 28.50; H, 4.57; N, 27.20%.

EXAMPLE 7

Hemifumarate salt of 3-amino-5-[2-[(2-guanidino-4-thiazolyl)methylthio]ethylamino]-4-methyl-1,2,4,6-thiatriazine 1,1-dioxide

Fine white crystals were obtained on recrystallisation from water, m.p. 161-166°C.

$\nu_{max}$ (Nujol mull) 3500, 3405, 3340, 3220 (broad), 1700, 1640, 1620, 1560, 1530, 1405, 1330, 1270, 1225, 1160, 1130, 1090, 1060, 985, 880, 855, 825, 800, 755, 720 and 680 $cm^{-1}$.

NMR: 2.62 (2H, t), 3.19 (3H, s), 3.35 (2H, m), 3.64 (2H, s), 6.60 (1H, s), 6.66 (1H, s), 7.08 (4H, very broad s, exchangeable), 7.35 (2H, broad s, exchangeable) and 7.64 (1H, t, exchangeable).

Microanalysis: Found: C, 30.56; H, 4.34; N, 27.37. $C_{12}H_{14}N_9O_4S_3.H_2O$ requires C, 30.84; H, 4.53; N, 26,96%.

EXAMPLE 8

Tartarate salt of 3-amino-5-[2-[(2-guanidino-4-thiazolyl)methylthio]ethylamino]-4-methyl-1,2,4,6-thiatriazine 1,1-dioxide

Fine white crysysals were obtained on recrystallisation from water, m.p. 136-140°C.

$\nu_{max}$ (Nujol mull) 3400, 3300 (broad), 3210, 1690, 1620, 1565, 1535 (sh), 1400, 1330, 1270, 1240, 1210, 1145, 1120, 1060, 1010, 990, 890, 860, 800 and 720 $cm^{-1}$.

NMR: 2.62 (2H, t), 3.19 (3H, s), 3.35 (2H, m), 3.63 (2H, s); 4.26 (2H, s), 6.66 (1H, s), 7.04 (4H, very broad s, exchangeable), 7.34 (2H, broad s, exchangeable), 7.64 (1H, t, exchangeable).

Microanalysis: Found: C, 29.74; H, 4.40; N, 22.69.
$C_{14}H_{20}N_9O_8S_3.H_2O$ requires C, 30.04; H, 4.50; N,22.53.

EXAMPLE 9

Picrate salt of 3-amino-5-[2-[(2-guanidino-4-thiazolyl)methylthio]ethylamino]-4-methyl-1,2,4,6-thiatriazine 1,1-dioxide

Yellow needles m.p. 241-244°C.

$\nu_{max}$ (Nujol mull) 3420, 3160 (broad), 1680, 1650, 1615, 1570, 1555, 1340, 1320, 1280, 1220, 1150 (broad), 1080, 980, 940, 910, 850, 790 and 720 $cm^{-1}$.

NMR: 2.61 (2H, t), 3.20 (2H, s), 3.36 (obscured by $H_2O$), 3.78 (2H, s), 7.24 (1H, s), 7.37 (2H, broad s, exchangeable), 7.67 (1H, t), 8.17 (4H, broad s, exchangeable) and 8.60 (2H, s).

Microanalysis: Found: C, 30.07; H, 3.47; N, 26.31.
$C_{16}H_{20}N_{12}O_9S_3.H_2O$ requires C, 30.09; H, 3.47; N, 26.32%.

Example A

3-N-[2-[(2-Guanidino-4-thiazolyl)methylthio]-ethyl]-amino-4-methyl-5-amino-1,2,4,6-thiatriazine-1,1-dioxide

To a solution of 4-methyl-3-(4-methylphenoxy)-5-amino-1,2,4,6-thiatriazine-1,1-dioxide (100 mg) in 5 ml of methanol was added a solution of 2-guanidino-4-[(2-aminoethyl)thiomethyl]thiazole (90 mg) in 10 ml of ethanol. The resulting solution was kept at room temperature for 16 hours then refluxed for 4 hours. The solvent was evaporated under vacuum and the residual solid chromatographed on silica gel, eluting with a chloroform/methanol/30 % aqueous ammonia mixture to give the title compound as a colourless solid (45 mg).

$^1$H n.m.r. (60 MHz) DMSO δ 2.62 (2 H,t), 3.20 (3 H,S), 3.34 (2H,t), 3.61 (2 H,S), 6.54 (1 H,S), 6.81 (4 H,br), 7.44 (1 H,br), 7.60 (1 H,br).

Example B

3-(4-methylphenoxy)-4-methyl-5-amino-1,2,4,6-thiatriazine-1,1-dioxide

A suspension of 5-amino-3-(4-methylphenoxy) 4 H-1,2,4,6-thiatriazine-1,1,-dioxide (508 mg) in 10 ml of methanol was added to a stirred solution of sodium (46 mg) in 20 ml of methanol and the mixture stirred for 15 minutes. The resulting solution was evaporated to dryness and the residual salt suspended in acetonitrile (25 ml). Methyliodide was then added to the stirred suspension and the mixture refluxed for 3 hours, before evaporating to dryness under vacuum. The solid residue was taken up in water and extracted once with ethyl acetate. The ethyl

acetate layer was dried and evaporated to a foam which was further purified by chromatography on silica gel, eluting with chloroform/methanol, to give the title compound (120 mg). m.p. 278 - 280°C.

'H n.m.r. (60 MHz) DMSO $\delta$ 2.18 (3 H,S), 3.27 (3 H,S), 7.05 (4 H,ABq), 7.60 (2 H,br).

Example C

3,5-Bis-(4-methylphenoxy)-4-methyl-1,2,4,6-thiatriazine-1-oxide

To freshly prepared 4-methylphenylcyanate (13.3 g) was added bis (trimethylsilyl) methylamine (8.7 g) at room temperature in an inert atmosphere. The resulting mixture was kept at this temperature for 70 hours in a sealed flask. The yellow oil thus obtained was decanted from a small amount of white solid and dissolved in dichloromethane (25 ml), cooles to -5°C and stirred whilst thionyl chloride (2.0 ml) was added dropwise over 20 mins, not allowing the temperature to exceed 10°C. The solution was then stirred for 1 hr. at 10°C. Iced water (10 ml) was cautiously added and the layers separated, the organic phase washed with water (10 ml), dried ($MgSO_4$) and evaporated to give an oil. Trituration with ether affords 4-methyl-3,5-di-4'-methylphenoxy-1,2,4,6-thiatriazine-1-oxide as a white solid 3.82 g. Crystallisation from ethanol gave an analytically pure sample as needles m.p. 231-3°C.

$\gamma_{max}$($CHCl_3$) 1670(s), 1396(s), 1368(s), 1172(m), 1105(m) $cm^{-1}$

$^1$H n.m.r. (60 MHz) $CHCl_3$, $\delta$ 2.31 (6 H,s), 3.62 (3 H,s), 7.00 (8 H,ABq)

Example D

3,5-Bis-(4-methylphenoxy)-4-methyl-1,2,4,6-thiatriazine-1,1-dioxide

To a stirred solution of 4-methyl,3,5-di-4'-methylphenoxy-1,2,4,6-thiatriazine-1-oxide (5.0 g) in chloroform (20 ml) at room temperature a solution of m-chloroperbenzoic acid (3.5 g) in chloroform (10 ml) was added over 1 - 2 minutes. The exothermic reaction heats the mixture to reflux after a few minutes (cooling may be required) and a white solid precipitates. After heating at reflux for an additional 5 minutes the solid was filtered off and washed with a little cold chloroform to afford the title compound 4.4 g, m.p. 288 - 289°C.

$\gamma_{max}$ 1690(s), 1673(s), 1380(m), 1160(m), 831(m), 823(m) $cm^{-1}$

$^{1}H$ n.m.r. (60 MHz) DMSO $\delta$ 2.25 (6 H,S), 3.54 (3 H,S), 7.08 (8 H,ABq)

Example E

3-N-/2-/(2-Guanidino-4-thiazolyl)methylthio/ethyl/amino-4-methyl-5-(4-methylphenoxy)-1,2,4,6-thiatriazine-1,1-dioxide

To a stirred solution of 4-methyl,3,5-di-4'-methylphenoxy-1,2,4,6-thiatriazine-1,1-dioxide (4.4 g) in acetonitrile (25 ml) was added 2-guanidino-4-/(2-aminoethyl)thiomethyl/-thiazole (2.8 g) as a solution in ethanol (10 ml). The resulting mixture was stirred at room temperature for 6 hours, evaporated to dryness and chromatographed on silica gel. Elution with a chloroform/methanol/30 % aqueous ammonia mixture gave the title compound as a solid (4.4 g) m.p. 194 - 6°C.

'H n.m.r. (250 MHz) DMSO δ 2.33 (3 H,S), 2.67 (2 H,t), 3.43 (5 H,m), 3.67 (2 H,S), 6.71 (1 H,S), 7.15 - 7.29 (8 H, m inc 4 H,ex), 8.18 (1 H,t,ex).

Example F

(i) 1-Phenyl-2-methyl-3-(N'-acetyl)sulphamoyl isothiourea

2.23 g of 1-acetyl-1-phenyl-2-methylisothiourea (See: H. Wheeler, Am. Chem. J., 27, 270 (1902) was dissolved in 50 ml of dry acetonitrile and stirred at room temperature. 1.25 g of triethylamine in 10 ml of dry acetonitrile and 1.47 g of aminosulphonyl chloride in 10 ml of dry acetonitrile were added dropwise simultaneously. The reaction was left to stir for 30 minutes, then evaporated to dryness in vacuo. The residue was partitioned between dichloromethane and water, and the aqueous layer extracted once again with dichloromethane. The combined organic layers were washed once with water, dried over $MgSO_4$ and evaporated, to give the title compound as a white crystalline solid: 2.8 g.

m.p. 144-148°C.

'H n.m.r. (250 MHz) $CDCl_3$, δ, 2.18 (s, 3H), 2.36 (s. 3H), 7.32-7.45 (m, 5H).

Using this procedure the following were obtained:

(2( 1,2-Dimethyl-3-(N'-benzoyl)sulphamoyl isothiourea

'H n.m.r. (60 MHz) $CDCl_3$, δ, 2.37 (s, 3H), 3.10 (d, 3H), 7.3-8.1 (m, 5H).

The n.m.r. and thin layer chromatography of the crude product from this preparation indicated the presence of some cyclized thiatriazine. The crude material was converted cleanly to the thiatriazine by heating according to example M.

(3) 1,2-Dimethyl-3-(N'-acetyl)-sulfamoyl isothiourea

'H n.m.r. (60 MHz) $CDCl_3$, δ, 2.14 (3H, s), 2.44 (3H, s), 3.06 (3H, d).

Example G

1-Benzoyl-1,2-dimethylisothiourea

2.32 g of N,S-dimethyl isothiourea hydriodide salt was stirred in suspension in 40 ml of tetrahydrofuran at -20°C. 2.1 g of triethylamine was added, followed by dropwise addition of 1.4 g of benzoyl chloride. The reaction was stirred for 30 minutes at -20°C and then allowed to warm to ambient. The solvent was evaporated in vacuo and the residue purified by column chromatography on silicagel, eluting with ethyl acetate-hexane to yield 1.1 g of the title compound as a gum.

'H n.m.r. (60 MHz) $CDCl_3$, δ, 2.23 (s, 3H), 3.33 (s, 3H), 7.1-7.6(m, 5H)

The isomeric 1,2-dimethyl-3-benzoylisothiourea was obtained as a 1:1 mixture with the above by carrying out the above procedure at ambient temperature in dichlormethane as solvent:

$^1$H n.m.r. (60 MHz) $CDCl_3$, $\delta$, 2.62 (s, 3H), 3.02 (d, 3H), 7.15-8.30 (m, 5H).

1-Acetyl-1,2-dimethylisothiourea

$^1$H n.m.r. (60 MHz) DMSO, $\delta$, 2.48 (3H, s), 2.66 (3H, s), 3.45 (3H, s).

CLAIMS:

1. A salt of 3-amino-5-[2-[(2-guanidino-4-thiazolyl)-methylthio]ethylamino]-4-methyl-1,2,4,6-thiatriazine-1,1-dioxide with an inorganic or an organic acid, with the exception of the hydrobromide salt.

2. A salt as claimed in claim 1, which salt is physiologically tolerable.

3. A salt as claimed in claim 2, with sulphuric acid or with hydrochloric acid.

4. A salt as claimed in claim 2, with a dicarboxylic acid.

5. A salt as claimed in claim 4, with maleic, fumaric, tartaric, oxalic or succinic acid.

6. 3-Amino-5-[2-[(2-guanidino-4-thiazolyl)methylthio]-ethylamino]-4-methyl-1,2,4,6-thiatriazine 1,1-dioxide or a salt thereof (with the exception of the hydrobromide salt), in crystalline form.

7. 3-Amino-5-[2-[(2-guanidino-4-thiazolyl)methylthio]-ethylamino]-4-methyl-1,2,4,6-thiatriazine-1,1-dioxide characterised by an infra-red spectrum (KBr disc) showing the following main peaks:
3480, 3380, 3600-2500 (very broad), 1640 (very broad), 1615, 1580, 1545, 1520, 1500, 1455, 1410, 1330, 1260, 1220, 1190, 1160, 1130, 1085, 1060, 1000, 850, 710 and 670 $cm^{-1}$.

8. A compound as claimed in claim 7, in the form of the dihydrate.

9. 3-Amino-5-[2-[(2-guanidino-4-thiazolyl)methylthio]-ethylamino]-4-methyl-1,2,4,6-thiatriazine-1,1-dioxide characterised by an infra-red spectrum (KBr disc) showing the following main peaks:
3580, 3420, 3340, 3180, 1660, 1610, 1570, 1530, 1520, 1455, 1410, 1365, 1340, 1270, 1250, 1220, 1190, 1130, 1120, 1075, 1010, 990, 970, 870, 860, 730, 700 and 660 $cm^{-1}$.

10. 3-Amino-5-[2-[(2-guanidino-4-thiazolyl)methylthio]-ethylamino]-4-methyl-1,2,4,6-thiatriazine-1,1-dioxide characterised by the following X-Ray Powder Diffraction Pattern expressed in terms of "d" spacings and relative intensities (I):

2.18 vw; 2.29 vw; 2.38 vw; 2.58 vw; 2.64 vw; 2.88 vw; 2.97 vw; 3.05 w; 3.17 w; 3.25 w; 3.33 w; 3.42 w; 3.50 w; 3.72 m; 4.07 m; 4.36 m; 5.10 m; 6.32 m; 6.74 s; 7.39 m.

11. A compound as claimed in claim 10 in the form of a monohydrate.

12. A hydrochloride salt of 3-amino-5-[2-[(2-guanidino-4-thiazolyl)methylthio]-ethylamino]-4-methyl-1,2,4,6-thiatriazine-1,1-dioxide characterised by an infra-red spectrum (KBr disc) showing the following main peaks: $\nu_{max}$ 3450-2500 (very broad), 3390, 3280, 3180, 3100, 3060, 1695, 1665, 1610, 1560, 1525, 1485, 1460, 1435, 1410, 1335, 1260, 1215, 1190, 1150, 1130, 1085, 1050, 980, 860, 720 and 650 $cm^{-1}$.

13. A hydrochloride salt of 3-amino-5-[2-[(2-guanidino-4-thiazolyl)methylthio]-ethylamino]-4-methyl-1,2,4,6-thiatriazine-1,1-dioxide characterised by the following X-Ray Powder Diffraction Pattern expressed in terms of "d" specings and relative intensities (I):

2.53 vw; 2.69 w; 2.83 w; 2.89 m; 2.94 m; 3.29 m; 3.52 m; 3.61 m; 3.92 s; 4.23 w; 4.38 s; 4.71 m; 5.24 m.

14. A hemisuccinate salt of 3-amino-5-[2-[(2-guanidino-4-thiazolyl)methylthio]ethylamino]-4-methyl-1,2,4,6-thiatriazine 1,1-dioxide characterised by an infra-red spectrum (KBr disc) showing the following main peaks: $\nu_{max}$ 3600-2500 (very broad), 1705, 1655 (sh), 1640 (sh), 1620, 1560-1530 (broad), 1490, 1460, 1440, 1405, 1385, 1330, 1240, 1220, 1195, 1150, 1120, 1090, 1060, 985, 855, 805, 715 and 660 $cm^{-1}$.

15. A hemisuccinate salt of 3-amino-5-[2-[(2-guanidino-4-thiazolyl)methylthio]ethylamino]-4-methyl-1,2,4,6-thiatriazine 1,1-dioxide characterised by the following X-Ray Powder Diffraction Pattern expressed in terms of "d" specings and relative intensities (I):

2.39 vw; 3.12 vw; 3.39 vw; 3.46 s; 3.62 m; 4.01 m; 4.11 m; 4.69 m; 5.26 w; 5.97 m; 6.76 m; 7.20 m; 7.99 m.

16. A salt as claimed in claim 14 in the form of the dihydrate.

17. A sulphate salt of 3-amino-5-[2-[(2-guanidino-4-thiazolyl)methylthio]ethylamino]-4-methyl-1,2,4,6-thiatriazine 1,1-dioxide sulphate characterised by an infra-red spectrum (KBr disc) showing the following main peaks: $\nu_{max}$ 3200-2500 (very broad), 1705, 1660, 1620, 1570, 1530, 1460, 1440, 1410, 1340, 1280, 1230, 1215, 1190, 1130, 1070, 1010, 990, 860, 810 and 715 $cm^{-1}$.

18. A salt as claimed in claim 17, having per molecule, half a molecule of sulphuric acid and one molecule of water as solvating agents.

19. A maleate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:
$\nu_{max}$ 3330 (broad), 3180, 3110, 1700, 1660, 1635, 1610, 1560, 1535, 1400, 1340, 1270, 1235, 1215, 1130, 1120, 1075, 1005, 995, 890, 865, 835, 750 and 720 $cm^{-1}$.

20. A hemi-oxalate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:
$\nu_{max}$ 3400 (broad), 3200 (broad), 1710, 1660, 1620, 1560, 1540, 1420, 1345, 1305, 1270 (broad), 1235, 1215, 1130 (broad), 1070, 1050, 1000, 980, 870, 840, 765, 725 and 715 $cm^{-1}$.

21. A salt as claimed in claim 20, having 1.5 molecules of water of hydration per molecule of the salt.

22. A hemifumarate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:
$\nu_{max}$ 3500, 3405, 3340, 3220 (broad), 1700, 1640, 1620, 1560, 1530, 1405, 1330, 1270, 1225, 1160, 1130, 1090, 1060, 985, 880, 855, 825, 800, 755, 720 and 680 $cm^{-1}$.

23. A salt as claimed in claim 22, having one molecule of water of hydration per molecule of salt.

24. A tartarate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:

$\nu_{max}$ 3400, 3300 (broad), 3210, 1690, 1620, 1565, 1535 (sh), 1400, 1240, 1210, 1330, 1270, 1240, 1145, 1120, 1060, 1010, 990, 890, 860, 800 and 720 $cm^{-1}$.

25. A salt as claimed in claim 24, having one molecule of water of hydration per molecule of the salt.

26. A compound or salt as claimed in any one of claims 7 to 25, in any hydrated and/or solvated form.

27. A tautomer of 3-amino-5-[2-[(2-guanidino-4-thiazolyl)-methylthio]-ethylamino]-4-methyl-1,2,4,6-thiatriazine-1,1-dioxide or a salt thereof as claimed in any one of claims 1 to 26.

28. A process for the production of a salt of 3-amino-5-[2-[(2-guanidino-4-thiazolyl)methylthio]ethylamino]-4-methyl-1,2,4,6-thiatriazine 1,1-dioxide, which comprises reacting 3-amino-5-[2-[(2-guanidino-4-thiazolyl)methylthio]ethylamino]-4-methyl-1,2,4,6-thiatriazine 1,1-dioxide with an acid.

29. A process for the production of 3-amino-5-[2-[(2-guanidino-4-thiazolyl)methylthio]ethylamino]-4-methyl-1,2,4,6-thiatriazine 1,1-dioxide and of salts thereof, in crystalline form, which comprises

a)i) reacting a 1,2,4,6-thiatriazine of formula A

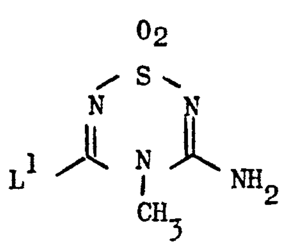

(A)

in which $L^1$ represents a halogen atom, an alkoxy or phenoxy group, an alkylthio or phenylthio group, or an alkylsulphonyl or phenylsulphonyl group, with a compound of

the formula B

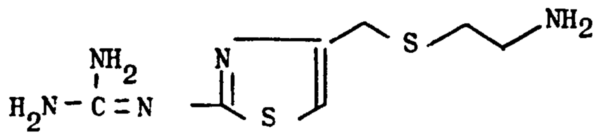 (B)

a)ii) reacting a 1,2,4,6-thiatriazine of formula C

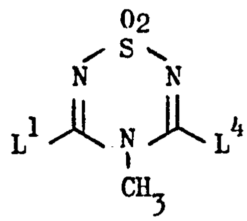 (C)

in which $L^1$ and $L^4$, which may be the same or different, each represents a halogen atom, an alkoxy or phenoxy group, an alkylthio or phenylthio group, or an alkylsulphonyl or phenylsulphonyl group, and $L^4$ may also represent a mono- or di- lower alkyl-substituted amine group, with a compound of formula B as defined above, and reacting the resulting compound of formula D

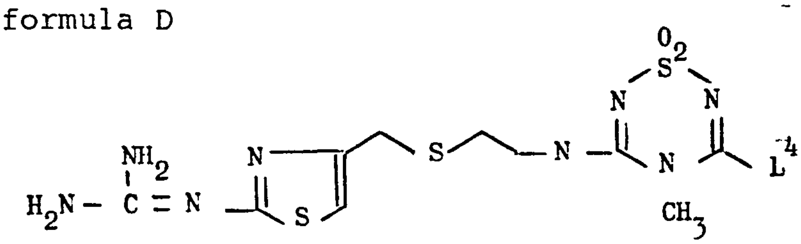 (D)

with ammonia; or

a)iii) reacting a 1,2,4,6-thiatriazine of the formula A with a compound of formula E

$$HS - CH_2 - CH_2 - NH_2 \qquad E$$

and reacting the resulting compound of formula F

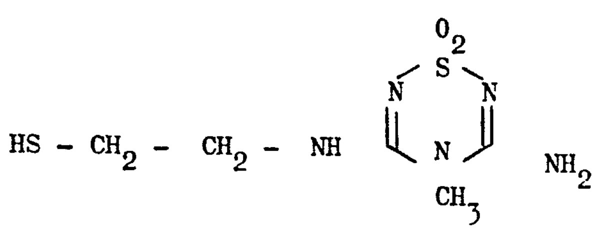 (F)

with a compound of the general formula G

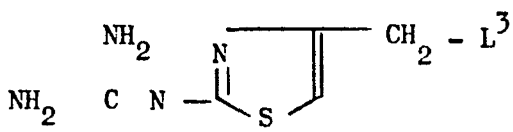 (G)

in which $L^3$ represents a leaving group, for example, a halogen atom, a hydroxy group, an alkoxy group, or a sulphonate ester, and isolating the resulting crystalline 3-amino-5-[2-[(2-guanidino-4-thiazolyl)methylthio]ethylamino]-4-methyl-1,2,4,6-thiatriazine 1,1-dioxide from the reaction mixture; or

b) converting 3-amino-5-[2-[(2-guanidino-4-thiazolyl)-methylthio]ethylamino]-4-methyl-1,2,4,6-thiatriazine 1,1-dioxide into a salt thereof, and isolating the resulting crystalline salt from the reaction mixture; or

c) converting a salt of 3-amino-5-[2-[(2-guanidino-4-thiazolyl)methylthio]ethylamino]-4-methyl-1,2,4,6-thiatriazine 1,1-dioxide into the free base, and isolating the resulting free base from the reaction mixture.

30. A process as claimed in claim 29, wherein a tautomer of a compound of formula A, C, D or F is used.

31. A process as claimed in claim 29 or claim 30, wherein the reaction mixture is cooled to obtain the product in crystalline form.

32. A process as claimed in any one of claims 29 to 31, wherein a resulting crystalline product is recrystallised.

33. A process as claimed in claim 32, wherein the solvent used for recrystallisation is a lower alcohol, a ketone, a mixture of any two or more thereof, or a mixture of any one or more thereof and water.

34. A process as claimed in claim 32, wherein the solvent is ethanol.

35. A process as claimed in claim 32, wherein the solvent used for recrystallisation is water.

36. A pharmaceutical preparation which comprises a compound or salt as claimed in any one of claims 1 to 27, in admixture or conjunction with a pharmaceutically suitable carrier.

37.A pharmaceutical preparation as claimed in claim 36, which also comprises a histamine H-1 antagonist.

38. Use of 3-amino-5-[2-[(2-guanidino-4-thiazolyl)-methylthioethylamino]-4-methyl-1,2,4,6-thiatriazine

1,1-dioxide or a physiologically tolerable salt thereof, as claimed in any one of claims 1 to 26 for the manufacture of a medicament for the treatment of those conditions resulting from stimulation by histamine of histamine H-2 receptors, either alone, or together with a histamine H-1 antagonist.

Claims for Austria:

. . A process for the production of a salt of 3-amino-5-[2-[(2-guanidino-4-thiazolyl)methylthio]ethylamino]-4-methyl-1,2,4,6-thiatriazine 1,1-dioxide, which comprises reacting 3-amino-5-[2-[(2-guanidino-4-thiazolyl)methylthio]ethylamino]-4-methyl-1,2,4,6-thiatriazine 1,1-dioxide with an acid.

2 . A process for the production of 3-amino-5-[2-[(2-guanidino-4-thiazolyl)methylthio]ethylamino]-4-methyl-1,2,4,6-thiatriazine 1,1-dioxide and of salts thereof, in crystalline form, which comprises

a)i) reacting a 1,2,4,6-thiatriazine of formula A

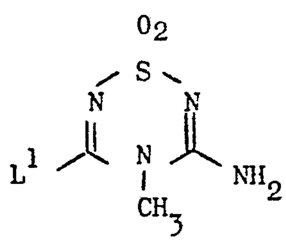

(A)

in which $L^1$ represents a halogen atom, an alkoxy or phenoxy group, an alkylthio or phenylthio group, or an alkylsulphonyl or phenylsulphonyl group, with a compound of the formula B

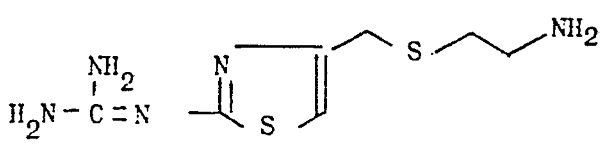

(B)

a)ii) reacting a 1,2,4,6-thiatriazine of formula C

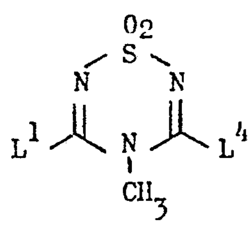

(C)

in which $L^1$ and $L^4$, which may be the same or different, each represents a halogen atom, an alkoxy or phenoxy group, an alkylthio or phenylthio group, or an alkylsulphonyl or phenylsulphonyl group, and $L^4$ may also represent a mono- or di- lower alkyl-substituted amine group, with a compound of formula B as defined above, and reacting the resulting compound of formula D

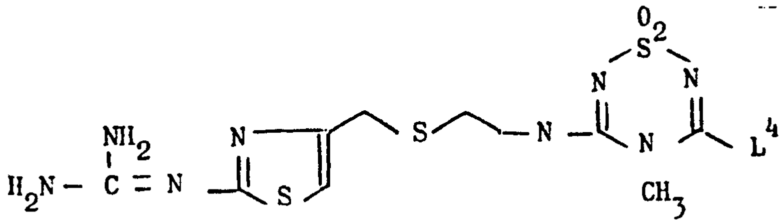

(D)

with ammonia; or

a)iii) reacting a 1,2,4,6-thiatriazine of the formula A with a compound of formula E

$HS - CH_2 - CH_2 - NH_2$ E

and reacting the resulting compound of formula F

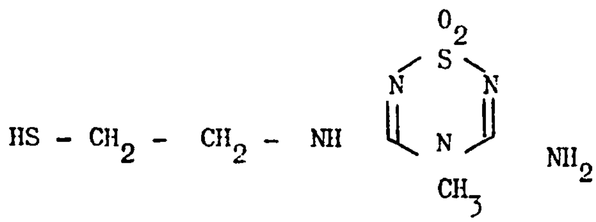

(F)

with a compound of the general formula G

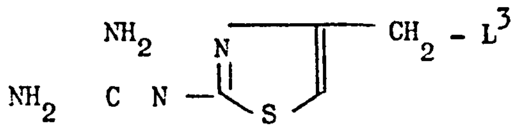

(G)

in which $L^3$ represents a leaving group, for example, a halogen atom, a hydroxy group, an alkoxy group, or a sulphonate ester, and isolating the resulting crystalline 3-amino-5-[2-[(2-guanidino-4-thiazolyl)methylthio]ethylamino]-4-methyl-1,2,4,6-thiatriazine 1,1-dioxide from the reaction mixture; or

b) converting 3-amino-5-[2-[(2-guanidino-4-thiazolyl)-methylthio]ethylamino]-4-methyl-1,2,4,6-thiatriazine 1,1-dioxide into a salt thereof, and isolating the resulting crystalline salt from the reaction mixture; or

c) converting a salt of 3-amino-5-[2-[(2-guanidino-4-thiazolyl)methylthio]ethylamino]-4-methyl-1,2,4,6-thiatriazine 1,1-dioxide into the free base, and isolating the resulting free base from the reaction mixture.

3. A process according to claim 1 in which a salt with sulphuric acid or hydrochloric acid is produced.

4. A process according to claim 1 in which a dicarboxylic acid is used.

5. A process according to claim 2 in which a 3-amino-5-[2-[(2-guanidino-4-thiazolyl)methylthio]-ethylamino]-4-methyl-1,2,4,6-thiatriazine 1,1-dioxide or a salt thereof (with the exception of the hydrobromide salt), in crystalline form is procuded.

6. A process according to claim 2 in which a 3-amino-5-[2-[(2-guanidino-4-thiazolyl)methylthio]-ethylamino]-4-methyl-1,2,4,6-thiatriazine-1,1-dioxide characterised by an infra-red spectrum (KBr disc) showing the following main peaks:
3480, 3380, 3600-2500 (very broad), 1640 (very broad), 1615, 1580, 1545, 1520, 1500, 1455, 1410, 1330, 1260, 1220, 1190, 1160, 1130, 1085, 1060, 1000, 850, 710 and 670 $cm^{-1}$ is produced.

7. A process as claimed in claim 6 in which a salt in the form of the dihydrate.

8. A process according to claim 2 in which a 3-amino-5-[2-[(2-guanidino-4-thiazolyl)methylthio]-ethylamino]-4-methyl-1,2,4,6-thiatriazine-1,1-dioxide characterised by an infra-red spectrum (KBr disc) showing the following main peaks:
3580, 3420, 3340, 3180, 1660, 1610, 1570, 1530, 1520, 1455, 1410, 1365, 1340, 1270, 1250, 1220, 1190, 1130, 1120, 1075, 1010, 990, 970, 870, 860, 730, 700 and 660 $cm^{-1}$ is produced.

9. A process according to claim 2 in which a 3-amino-5-[2-[(2-guanidino-4-thiazolyl)methylthio]-ethylamino]-4-methyl-1,2,4,6-thiatriazine-1,1-dioxide characterised by the following X-Ray Powder Diffraction Pattern expressed in terms of "d" spacings and relative intensities (I):

2.18 vw; 2.29 vw; 2.38 vw; 2.58 vw; 2.64 vw; 2.88 vw; 2.97 vw; 3.05 w; 3.17 w; 3.25 w; 3.33 w; 3.42 w; 3.50 w; 3.72 m; 4.07 m; 4.36 m; 5.10 m; 6.32 m; 6.74 s; 7.39 m is produced.

10. A process according to claim 2 in which a compound in the form of a monohydrate is produced.

11. A process according to claim 2 in which a hydrochloride salt of 3-amino-5-[2-[(2-guanidino-4-thiazolyl)methylthio]-ethylamino]-4-methyl-1,2,4,6-thiatriazine-1,1-dioxide characterised by an infra-red spectrum (KBr disc) showing the following main peaks: $\nu_{max}$ 3450-2500 (very broad), 3390, 3280, 3180, 3100, 3060, 1695, 1665, 1610, 1560, 1525, 1485, 1460, 1435, 1410, 1335, 1260, 1215, 1190, 1150, 1130, 1085, 1050, 980, 860, 720 and 650 $cm^{-1}$ is produced.

12. A process according to claim 2 in which a hydrochloride salt of 3-amino-5-[2-[(2-guanidino-4-thiazolyl)methylthio]-ethylamino]-4-methyl-1,2,4,6-thiatriazine-1,1-dioxide characterised by the following X-Ray Powder Diffraction Pattern expressed in terms of "d" specings and relative intensities (I):
2.53 vw; 2.69 w; 2.83 w; 2.89 m; 2.94 m; 3.29 m; 3.52 m; 3.61 m; 3.92 s; 4.23 w; 4.38 s; 4.71 m; 5.24 m is produced.

13. A process according to claim 2 in which a hemisuccinate salt of 3-amino-5-[2-[(2-guanidino-4-thiazolyl)methylthio]ethylamino]-4-methyl-1,2,4,6-thiatriazine 1,1-dioxide characterised by an infra-red spectrum (KBr disc) showing the following main peaks: $\nu_{max}$ 3600-2500 (very broad), 1705, 1655 (sh), 1640 (sh), 1620, 1560-1530 (broad), 1490, 1460, 1440, 1405, 1385, 1330, 1240, 1220, 1195, 1150, 1120, 1090, 1060, 985, 855, 805, 715 and 660 $cm^{-1}$ is produced.

14. A process according to claim 2 in which a hemisuccinate salt of 3-amino-5-[2-[(2-guanidino-4-thiazolyl)methylthio]ethylamino]-4-methyl-1,2,4,6-thiatriazine 1,1-dioxide characterised by the following X-Ray Powder Diffraction Pattern expressed in terms of "d"

specings and relative intensities (I):
2.39 vw; 3.12 vw; 3.39 vw; 3.46 s; 3.62 m; 4.01 m; 4.11 m; 4.69 m; 5.26 w; 5.97 m; 6.76 m; 7.20 m; 7.99 m is produced.

15. A process according to claim 2 in which a salt as claimed in claim 13 in the form of the dihydrate is produced.

16. A process according to claim 2 in which a sulphate salt of 3-amino-5-[2-[(2-guanidino-4-thiazolyl)methylthio]ethylamino]-4-methyl-1,2,4,6-thiatriazine 1,1-dioxide sulphate characterised by an infra-red spectrum (KBr disc) showing the following main peaks:
$\nu_{max}$ 3200-2500 (very broad), 1705, 1660, 1620, 1570, 1530, 1460, 1440, 1410, 1340, 1280, 1230, 1215, 1190, 1130, 1070, 1010, 990, 860, 810 and 715 $cm^{-1}$ is produced.

17. A process according to claim 16 in which a salt having per molecule half a molecule of sulphuric acid and one molecule of water as solvating agents is produced.

18. A process as claimed in claim 2 in which a maleate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:
$\nu_{max}$ 3330 (broad), 3180, 3110, 1700, 1660, 1635, 1610, 1560, 1535, 1400, 1340, 1270, 1235, 1215, 1130, 1120, 1075, 1005, 995, 890, 865, 835, 750 and 720 $cm^{-1}$ is produced.

19. A process as claimed in claim 2 in which a hemi-oxalate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:
$\nu_{max}$ 3400 (broad), 3200 (broad), 1710, 1660, 1620, 1560, 1540, 1420, 1345, 1305, 1270 (broad), 1235, 1215, 1130 (broad), 1070, 1050, 1000, 980, 870, 840, 765, 725 and 715 $cm^{-1}$ is produced.

20. A process according to claim 19 in which a salt having 1.5 molecules of water of hydration per molecule of the salt is produced.

21. A process according to claim 2 in which a hemifumarate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:

$\nu_{max}$ 3500, 3405, 3340, 3220 (broad), 1700, 1640, 1620, 1560, 1530, 1405, 1330, 1270, 1225, 1160, 1130, 1090, 1060, 985, 880, 855, 825, 800, 755, 720 and 680 $cm^{-1}$ is produced.

22. A process as claimed in claim 21 in which a salt having one molecule of water of hydration per molecule of salt is produced.

23. A process according to claim 2 in which a tartarate salt of 3-amino-5-[2-[(2-guanidino-4-thiazolyl)methylthio]ethylamino]-4-methyl-1,2,4,6-thiatriazine 1,1-dioxide characterised by an infra-red spectrum (mineral oil mull) showing the following main peaks:

$\nu_{max}$ 3400, 3300 (broad), 3210, 1690, 1620, 1565, 1535 (sh), 1400, 1240, 1210, 1330, 1270, 1240, 1145, 1120, 1060, 1010, 990, 890, 860, 800 and 720 $cm^{-1}$ is produced.

24. A process according to claim 23 in which a salt having one molecule of water of hydration per molecule of the salt is produced.

25. A process for the production of a pharmaceutical preparation which comprises admixing or conjoining a compound or salt as claimed in any one of claims 1 to 24 with a pharmaceutically suitable carrier.

26. A process for the production of a pharmaceutical preparation as claimed in claim 25, which also comprises a histamine H-1 antagonist.

FIG. 1

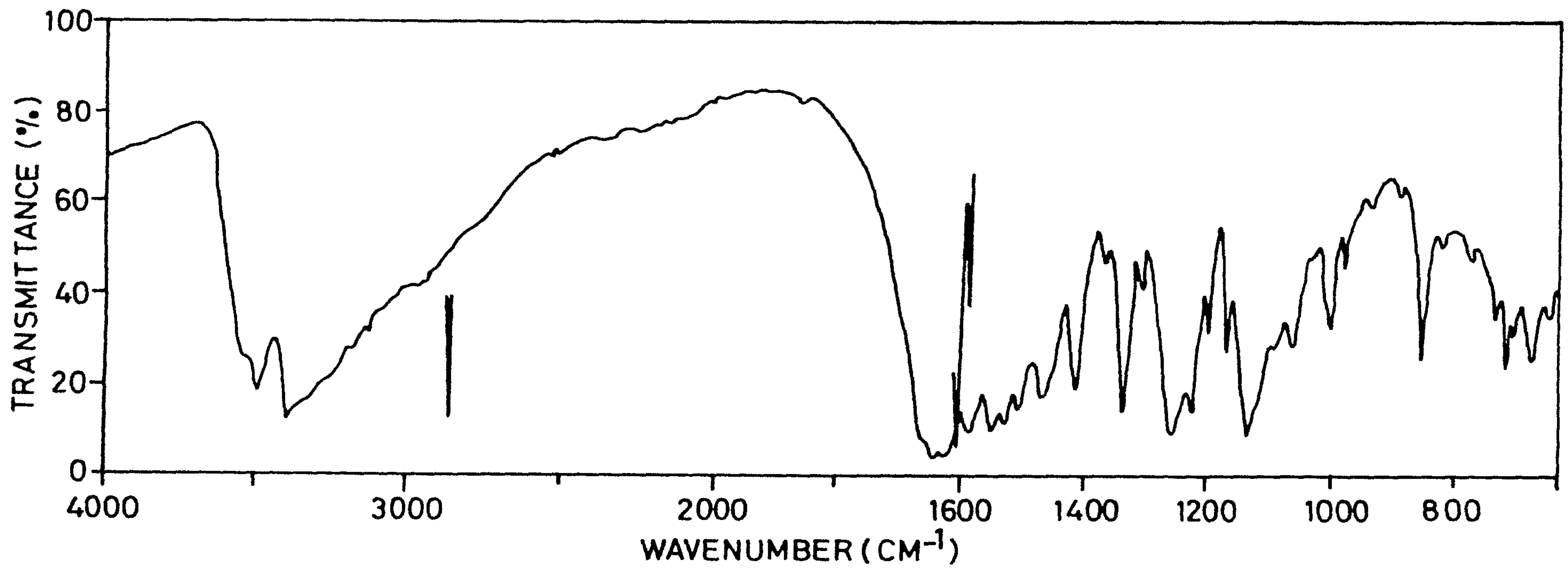
TRANSMITTANCE (%)
100
80
60
40
20
0
4000
3000
2000
1600
1400
1200
1000
800
WAVENUMBER (CM$^{-1}$)

FIG.2

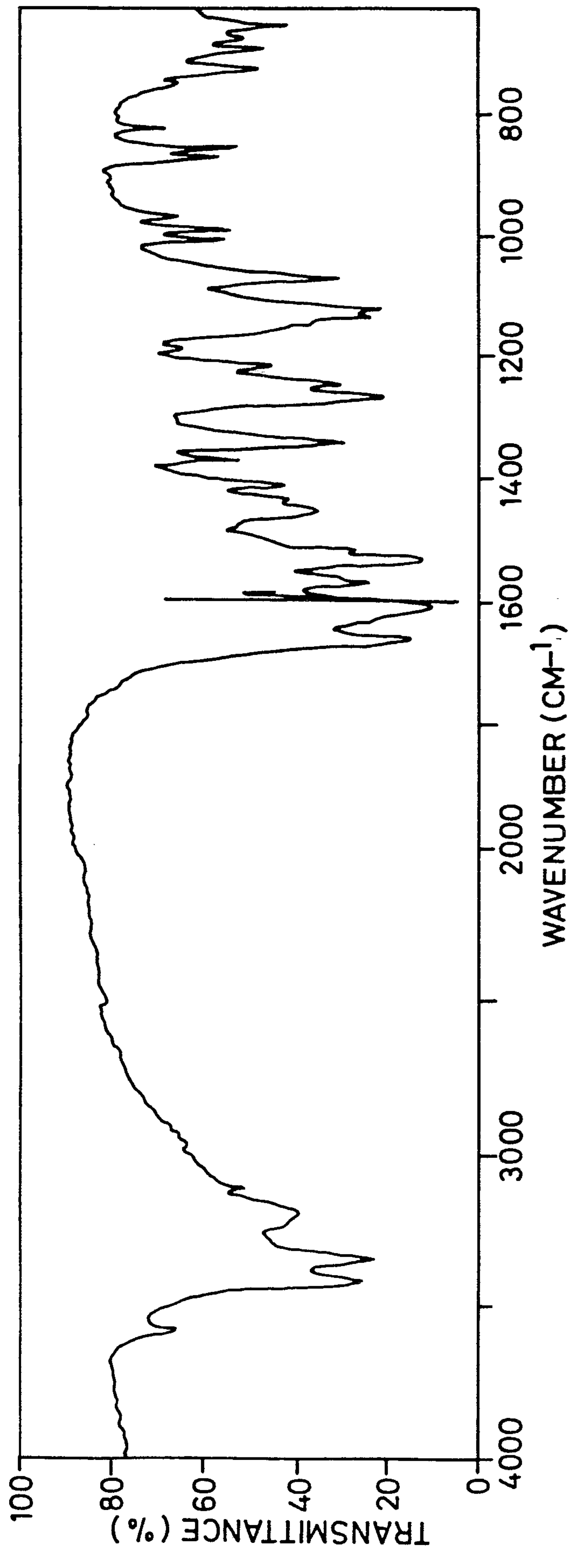
TRANSMITTANCE (%)
100
80
60
40
20
0
4000
3000
2000
1600
1400
1200
1000
800
WAVENUMBER (CM-1)

FIG. 3

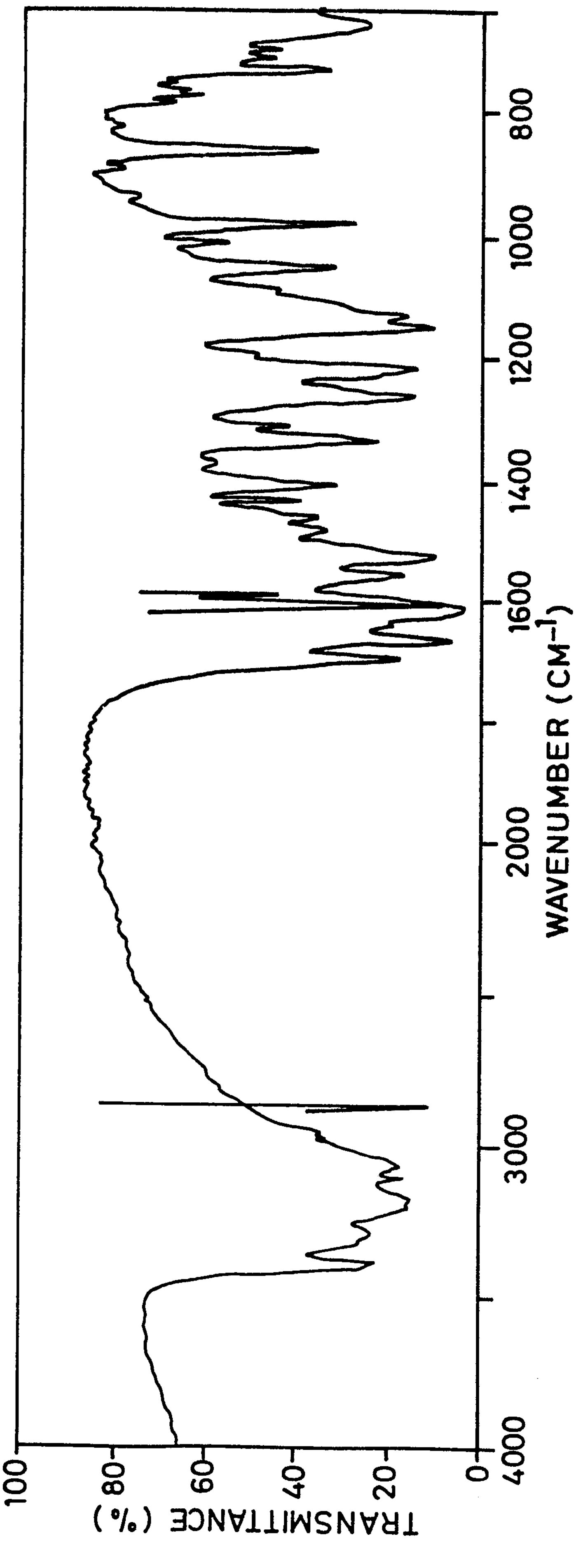
TRANSMITTANCE (%)
100
80
60
40
20
0
4000
3000
2000
1600
1400
1200
1000
800
WAVENUMBER (CM$^{-1}$)

FIG. 4

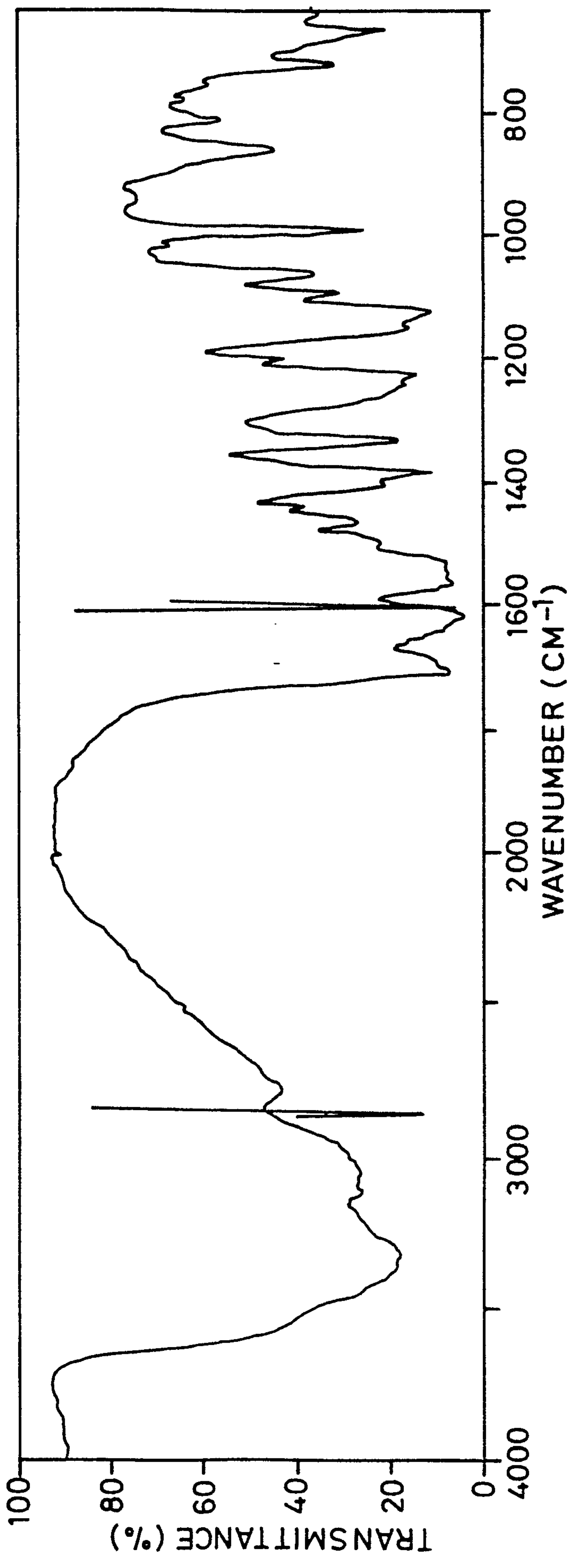

TRANSMITTANCE (%)
100
80
60
40
20
0
4000
3000
2000
1600
1400
1200
1000
800
WAVENUMBER (CM$^{-1}$)